# EUROPEAN PATENT SPECIFICATION

(11) **EP 4 013 420 B1**
(45) Date of publication and mention of the grant of the patent: **01.11.2023**
(21) Application number: 20764496.4
(22) Date of filing: 13.08.2020
(51) Int. Cl.: A61K 31/5383, A61K 31/7064, A61P 43/00

(54) **METHOD OF BLOCKING OR AMELIORATING CYTOKINE RELEASE SYNDROME**
VERFAHREN ZUR BLOCKIERUNG ODER LINDERUNG DES ZYTOKINFREISETZUNGSSYNDROMS
PROCÉDÉ DE BLOCAGE OU D'AMÉLIORATION DU SYNDROME DE LIBÉRATION DES CYTOKINES

(30) Priority: 14.08.2019 US 201962886806 P
(43) Date of publication of application: 22.06.2022
(73) Proprietor: Rigel Pharmaceuticals, Inc., South San Francisco, CA 94080 (US)
(72) Inventor: TAYLOR, Vanessa, South San Francisco California 94080 (US); ISSAKANI, Sarkiz, South San Francisco California 94080 (US); YOUNG, Chi, South San Francisco California 94080 (US)
(74) Representative: J A Kemp LLP
(86) International application number: PCT/US2020/046060
(87) International publication number: WO 2021/030526

(56) References cited:
- WO-A1-2008/064274
- WO-A1-2019/133245
- WO-A2-2007/120980
- WO-A2-2018/013918

## Description

### CROSS REFERENCE TO RELATED APPLICATION

This application claims the benefit under 35 U.S.C. § 119(e) of the earlier filing date of U.S. Provisional Application No. 62/886,806, filed on August 14, 2019.

### FIELD

The present application concerns compounds, and salts, solvates, N-oxides and/or prodrugs thereof, and pharmaceutical compositions containing them, for use in methods for treating and/or preventing cytokine release syndrome.

### BACKGROUND

Cytokine release syndrome (CRS) is a potentially life-threatening condition that may result from a variety of factors, including severe viral infections such as influenza, administration of antibodies that are used for immunotherapy, such as cancer immunotherapy, and non-protein-based cancer drugs such as oxaliplatin and lenalidomide. Immunotherapy can involve high levels of immune activation that exceed naturally occurring immune activation levels, and CRS is a non-antigen specific toxicity that can occur as a result. As immune-based therapies become more potent, CRS is becoming increasing diagnosed. CRS has also been observed in the setting of haploidentical donor stem cell transplantation, and graft-versus-host disease. Shimabukuro-Vornhagen et al., Journal for ImmunoTherapy of Cancer 6:56 (2018). CRS is associated with elevated circulating levels of several cytokines including interleukin (IL)-6 and interferon γ. Lee et al., Blood 124(2):188-195 (10 July 2014; Epub 29 May 2014).

CRS typically is clinically observed when significant numbers of lymphocytes and/or myeloid cells are activated and release inflammatory cytokines. The cytokine release may be induced by chemo- or biotherapy, and/or may be associated with therapeutic antibody treatments, such as immunotherapy, for example, for cancer treatment. Exemplary immunotherapies that may result in CRS include, but are not limited to, therapies where the cells express recombinant receptors, such as chimeric antigen receptors (CARs) and/or other transgenic receptors such as T cell receptors (TCRs). CRS induced by CAR T therapy generally occurs within days of T cell infusion at the peak of CAR T cell expansion. Giavridis et al., Nat Med. 24(6):731-738 (June 2018; Epub 28 May 2018). Examples of CAR T therapy that can induce CRS include axicabtagene ciloleucel (marketed as YESCARTA^{®}) and tisagenlecleucel (marketed as KYMRIAH^{®}).

Highly elevated interleukin 6 (IL-6) levels have been observed in patients with CRS and also in murine models of the disease, indicating that IL-6 may have a role in CRS pathophysiology. Shimabukuro-Vomhagen, J Immunother Cancer 6(1), 56 (2018). IL-6 can signal via two different modes. Classical IL-6 signaling involves binding of IL-6 to a membrane-bound IL-6 receptor. However, the IL-6 receptor does not possess intracellular signaling domains. Instead, after soluble IL-6 binds to membrane-bound IL-6 receptors, the IL-6/IL-6 receptor complex binds to membrane-bound gp130, which initiates signaling through its intracellular domain. In trans-signaling, IL-6 binds to a soluble form of the IL-6 receptor, which is typically cleaved from the cell surface by metalloproteinases. The resulting soluble IL-6/IL-6 receptor complex binds to gp130 and therefore can also induce signaling in cell types that do not express membrane bound IL-6 receptors.

IL-6 contributes to many of the key symptoms of CRS. Via trans-signaling, IL-6 leads to characteristic symptoms of severe CRS, i.e. vascular leakage, and activation of the complement and coagulation cascade inducing disseminated intravascular coagulation (DIC). In addition, IL-6 likely contributes to cardiomyopathy that is often observed in patients with CRS by promoting myocardial dysfunction. In a murine model, CRS developed within 2-3 days of CAR T cell infusion and could be lethal. Giavridis et al., Nat Med. 24(6): 731-738 (2018). CRS symptoms may start within minutes or hours of the start of antibody treatment, and can include a fever, which may reach or exceed 40 °C, nausea, fatigue, headache, tachycardia, hypotension, rash, shortness of breath, and/or myalgias. However, in certain cases, additional and potentially more serious complications may develop, including cardiac dysfunction, adult respiratory distress syndrome, neurological toxicity, renal and/or hepatic failure, and/or disseminated intravascular coagulation.

The National Cancer Institute Common Terminology Criteria for Adverse Events (CTCAE v. 5.0, pub. November 27, 2017) includes a grading system for CRS.
Grade 1: Fever with or without constitutional symptoms.
Grade 2: Hypotension responding to fluids; hypoxia responding to <40% O2.
Grade 3: Hypotension managed with one pressor; hypoxia requiring ≥ 40% O2.
Grade 4: Life-threatening consequences; urgent intervention indicated.
Grade 5: Death

WO 2019/133245 relates to methods and materials for preventing CRS. Methods and materials for using one or more catecholamine inhibitors to prevent a mammal from developing CRS are provided.

WO 2018/013918 concerns compositions and methods for treating diseases associated with expression of an antigen or for treating or preventing CRS, e.g. by administering a CAR therapy with a kinase inhibitor such as a JAK-STAT inhibitor and/or a BTK inhibitor.

WO 2008/064274 relates to prodrugs of biologically active 2,4-pyrimidinediamine compounds, salts and hydrates of those prodrugs; compositions comprising the prodrugs; methods of synthesizing the prodrugs and methods of using the prodrugs.

WO 2007/120980 provides 3-hydroxyphenyl-2,4-pyrimidinediamine compounds and related compositions and methods for treating a variety of autoimmune diseases.

### SUMMARY

The invention is as defined in the claims. Accordingly, the invention provides a compound for use in a method for treating and/or preventing cytokine release syndrome (CRS) in a subject experiencing, or at risk of developing, CRS, wherein the compound has a structure according to Formula I as defined in claim 1.

It should be noted, for the avoidance of doubt, that any disclosed references to methods of medical treatment using the compounds are not part of the invention, but in the context of the invention should instead be interpreted as the compounds for use in the relevant methods of medical treatment.

Thus, described herein is a method for treating or preventing CRS. The method comprises administering to a subject experiencing CRS, or at risk of developing CRS, an effective amount of a compound. The compound may be a kinase modulator and/or inhibitor, such as a spleen tyrosine kinase (Syk) modulator and/or inhibitor. The compound has a structure according to Formula I or a salt, solvate, N-oxide and/or prodrug thereof. 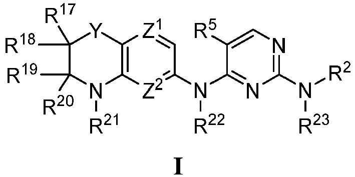 With respect to Formula I, Y is selected from CH₂, NR²⁴, O, S, S(O) and S(O)₂. Z¹ and Z² are each, independently of one another, selected from CH and N. R² is selected from (C₁-C₈) alkyl optionally substituted with one or more of the same or different R⁸ groups, (C₃-C₈) cycloalkyl optionally substituted with one or more of the same or different R⁸ groups, cyclohexyl optionally substituted with one or more of the same or different R⁸ groups, 3-8 membered heterocycloalkyl optionally substituted with one or more of the same or different R⁸ groups, (C₆-C₁₄)aryl optionally substituted with one or more of the same or different R⁸ groups, phenyl optionally substituted with one or more of the same or different R⁸ groups and 5-15 membered heteroaryl optionally substituted with one or more of the same or different R⁸ groups. R⁵ is selected from halo, cyano, nitro, or trihalomethyl. Each R⁸ independently is selected from R^{a}, R^{b}, R^{a} substituted with one or more of the same or different R^{a} or R^{b}, -OR^{a} substituted with one or more of the same or different R^{a} or R^{b}, -B(OR^{a})₂, -B(NR^{c}R^{c})₂, -(CH₂)*ₘ*-R^{b}, -(CHR^{a})*ₘ*-R^{b}, -O-(CH₂)*ₘ*-R^{b}, -S-(CH₂)*ₘ*-R^{b}, -O-CHR^{a}R^{b}, -O-CR^{a}(R^{b})₂, -O-(CHR^{a})*ₘ*-R^{b}, -O- (CH₂)*ₘ*-CH[(CH₂)*ₘ*R^{b}]R^{b}, -S-(CHR^{a})*ₘ*-R^{b}, -C(O)NH-(CH₂)*ₘ*-R^{b}, -C(O)NH-(CHRa)*ₘ*-R^{b}, -O-(CH₂)*ₘ*-C(O)NH-(CH₂)*ₘ*-R^{b}, -S-(CH₂)*ₘ*-C(O)NH-(CH₂)*ₘ*-R^{b}, -O-(CHR^{a})*ₘ*-C(O)NH-(CHR^{a})*ₘ*-R^{b}, -S-(CHR^{a})*ₘ*-C(O)NH-(CHR^{a})*ₘ*-R^{b}, -NH-(CH₂)*ₘ*-R^{b}, -NH-(CHR^{a})*ₘ*-R^{b}, -NH[(CH₂)*ₘ*R^{b}], -N[(CH₂)*ₘ*R^{b}]₂, -NH-C(O)-NH-(CH₂)*ₘ*-R^{b}, -NH-C(O)-(CH₂)*ₘ*-CHR^{b}R^{b} and -NH-(CH₂)*ₘ*-C(O)-NH-(CH₂)*ₘ*-R^{b}.

R¹⁷ is selected from hydrogen, halogen, or (C₁-C₈) alkyl, R¹⁸ is selected from hydrogen, halogen, (C₁-C₈) alkyl, or, alternatively, R¹⁸ may be taken together with R¹⁷ to form an oxo (=O) group or, together with the carbon atom to which they are attached, a spirocycle containing from 3 to 7 carbon atoms. R¹⁹ is selected from hydrogen, or (C₁-C₈) alkyl, R²⁰ is selected from hydrogen, or (C₁-C₈) alkyl, or, alternatively, R²⁰ may be taken together with R¹⁹ to form an oxo (=O) group or, together with the carbon atom to which they are attached, a spirocycle containing from 3 to 7 carbon atoms.

R²¹, R²² and R²³ are each, independently of one another, selected from hydrogen or phosphonooxyalkyl. R²⁴ is selected from hydrogen, (C₁-C₈) alkyl, or phosphonooxyalkyl. In some embodiments, at least one, such as 1, 2, 3, or 4, of R²¹, R²², R²³ and R²⁴ is phosphonooxyalkyl. In some embodiments, exactly one of R²¹, R²², R²³ and R²⁴ is phosphonooxyalkyl, and in certain embodiments, R²¹ is phosphonooxyalkyl. In some embodiments, at least one, such as 1, 2, 3, or 4, of R²¹, R²², R²³ and R²⁴ is hydrogen. In some embodiments, three of R²¹, R²², R²³ and R²⁴ are hydrogen, and in particular embodiments, R²¹, R²², R²³ and R²⁴ are all hydrogen. In other embodiments, R²¹ is phosphonooxyalkyl and R²², R²³ and R²⁴ are hydrogen.

Each R^{a} is, independently of the others, selected from hydrogen, (C₁-C₈) alkyl, (C₃-C₈) cycloalkyl, cyclohexyl, (C₄-C₁₁) cycloalkylalkyl, (C₆-C₁₀) aryl, phenyl, (C₇-C₁₆) arylalkyl, benzyl, 2-6 membered heteroalkyl, 3-8 membered heterocycloalkyl, 4-11 membered heterocycloalkylalkyl, 5-10 membered heteroaryl and 6-16 membered heteroarylalkyl. Each R^{b} is independently selected from =O, -OR^{a}, (C₁-C₃) haloalkyloxy, =S, -SR^{a}, =NR^{a}, =NOR^{a}, -NR^{c}R^{c}, halogen, -CF₃, -CN, -NC, -OCN, -SCN, -NO, -NO₂, =N₂, -N₃, -S(O)R^{a}, -S(O)₂R^{a}, -S(O)₂OR^{a}, -S(O)NR^{c}R^{c}, -S(O)₂NR^{c}R^{c}, -OS(O)R^{a}, -OS(O)₂R^{a}, -OS(O)₂OR^{a}, -OS(O)₂NR^{c}R^{c}, -C(O)R^{a}, -C(O)OR^{a}, -C(O)NR^{c}R^{c}, -C(NH)NR^{c}R^{c}, -C(NR^{a})NR^{c}R^{c}, -C(NOH)R^{a}, -C(NOH)NR^{c}R^{c}, -OC(O)R^{a}, -OC(O)OR^{a}, -OC(O)NR^{c}R^{c}, -OC(NH)NR^{c}R^{c}, -OC(NR^{a})NR^{c}R^{c}, -[NHC(O)]*ₙ*R^{a}, -[NR^{a}C(O)]*ₙ*R^{a}, -[NHC(O)]*ₙ*OR^{a}, -[NR^{a}C(O)]*ₙ*OR^{a}, -[NHC(O)]*ₙ*NR^{c}R^{c}, -[NR^{a}C(O)]*ₙ*NR^{c}R^{c}, -[NHC(NH)]*ₙ*NR^{c}R^{c} or -[NR^{a}C(NR^{a})]*ₙ*NR^{c}R^{c}. Each R^{c} is, independently of the others, selected from R^{a} or an amino-protecting group selected from formyl, acetyl, trifluoroacetyl, benzyl, benzyloxycarbonyl, *tert-*butoxycarbonyl, trimethylsilyl, 2-trimethylsilyl-ethanesulfonyl, trityl and substituted trityl groups, allyloxycarbonyl, 9-fluorenylmethyloxycarbonyl, or nitro-veratryloxycarbonyl, or, alternatively, two R^{c}s bonded to the same nitrogen atom are taken together with that nitrogen atom to form a 5 to 8-membered heterocycloalkyl or heteroaryl which may optionally include one or more of the same or different additional heteroatoms and which may optionally be substituted with one or more of the same or different R^{a} groups.

Each *m* is, independently of the others, an integer from 1 to 3, and/or each *n* is, independently of the others, an integer from 0 to 3.

In some embodiments, the compound has a formula selected from 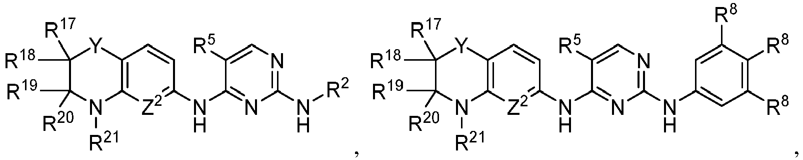 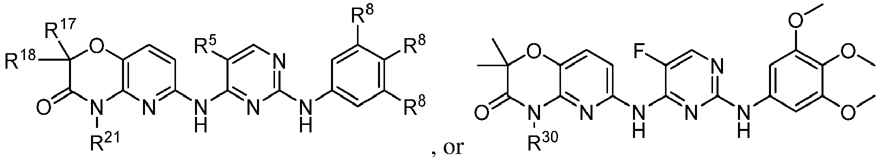 or a salt, solvate, N-oxide or prodrug thereof, where R³⁰ is H or phosphonooxyalkyl. And in certain embodiments, the compound is 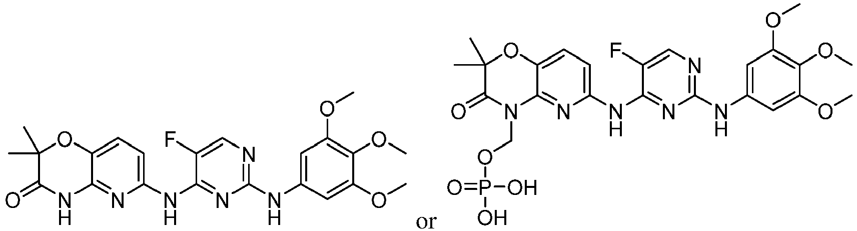 or a salt and/or solvate thereof, such as

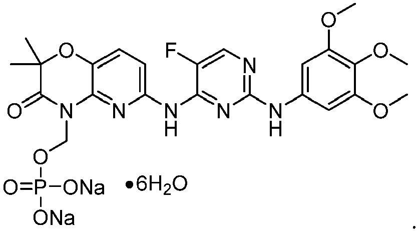

In any embodiments of the method, the subject may not exhibit a sign or symptom of CRS and/or may be at risk of developing CRS. In such embodiments, administering the compound substantially prevents the onset of CRS, or prevents the onset of grade 2 or higher CRS.

In other embodiments, the subject exhibits at least one sign or symptom of CRS and may exhibit at least one sign or symptom of grade 1 CRS. Alternatively, the subject may exhibit at least one sign or symptom of grade 2 or higher CRS, such as grade 3 or higher CRS. The compound may be administered within 24 hours of the onset of the sign or symptom, and/or administering the compound may ameliorate the sign or symptom of CRS, compared to the severity of the sign or symptom prior to administration of the compound, such as reducing the grade of CRS from 4 to 3, 2 or 1, or from 3, to 2 or 1, or from 2 to 1. Alternatively, CRS symptoms are substantially reduced to below grade 1 level, such that the subject no longer experiences symptoms associated with CRS. In some embodiments the sign or symptom is a fever and may be a fever of 40 °C or higher.

The method may comprise administering to a subject that has previously be administered a first therapy for which CRS is a known, suspected, or potential side effect. Administration of the first therapy may be initiated from greater than zero to 10 days prior to administration of the compound. Alternatively, the compound may be administered to a subject who will be, or is concurrently being, administered a first therapy for which CRS is a known, suspected, and/or potential side effect. In any embodiments, the first therapy may comprise a cell therapy, including, but not limited to, chimeric antigen receptor (CAR)-expressing therapy and/or a transgenic receptor therapy. Cell-free antibodies are also known to elicit this syndrome, particularly those that activate T-cells, including, but not limited to, CAMPATH 1-H, blinatumomab, and/or rituximab.

In some embodiments, the method may further comprise administering a second therapeutic agent, for example, a steroid, an anti-inflammatory agent, an immunosuppressant, or a combination thereof. The steroid may be a corticosteroid, such as, for example, dexamethasone or prednisone, or a combination thereof. In any embodiments, the compound may be administered substantially simultaneously with the second therapeutic agent, or the compound and second therapeutic agent may be administered sequentially in any order.

The foregoing and other objects, features, and advantages of the technology will become more apparent from the following detailed description.

### DETAILED DESCRIPTION

### I. Definitions

The following explanations of terms and methods are provided to better describe the present disclosure and to guide those of ordinary skill in the art in the practice of the present disclosure. The singular forms "a," "an," and "the" refer to one or more than one, unless the context clearly dictates otherwise. The term "or" refers to a single element of stated alternative elements or a combination of two or more elements, unless the context clearly indicates otherwise. As used herein, "comprises" means "includes." Thus, "comprising A or B," means "including A, B, or A and B," without excluding additional elements.

Unless otherwise indicated, all numbers expressing quantities of components, molecular weights, percentages, temperatures, times, and so forth, as used in the specification or claims are to be understood as being modified by the term "about." Accordingly, unless otherwise indicated, implicitly or explicitly, the numerical parameters set forth are approximations that may depend on the desired properties sought and/or limits of detection under standard test conditions/methods. When directly and explicitly distinguishing embodiments from discussed prior art, the embodiment numbers are not approximates unless the word "about" is recited.

Unless explained otherwise, all technical and scientific terms used herein have the same meaning as commonly understood to one of ordinary skill in the art to which this disclosure belongs. Although methods and materials similar or equivalent to those described herein can be used in the practice or testing of the present disclosure, suitable methods and materials are described below. The materials, methods, and examples are illustrative only and not intended to be limiting.

When chemical structures are depicted or described, unless explicitly stated otherwise, all carbons are assumed to include hydrogen so that each carbon conforms to a valence of four. For example, in the structure on the left-hand side of the schematic below there are nine hydrogen atoms implied. The nine hydrogen atoms are depicted in the right-hand structure.

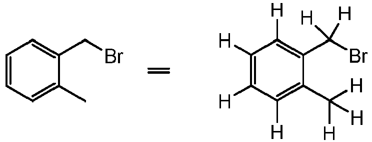

Sometimes a particular atom in a structure is described in textual formula as having a hydrogen or hydrogen atoms, for example -CH₂CH₂-. It will be understood by a person of ordinary skill in the art that the aforementioned descriptive techniques are common in the chemical arts to provide brevity and simplicity to description of organic structures.

If a group R is depicted as "floating" on a ring system, as for example in the group: 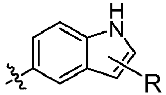 then, unless otherwise defined, a substituent R can reside on any atom of the fused bicyclic ring system, so long as a stable structure is formed that conforms to standard valence conditions as understood by a person of ordinary skill in the art. In the example depicted, the R group can reside on an atom in either the 5-membered or the 6-membered ring of the indolyl ring system, including the heteroatom by replacing the explicitly recited hydrogen, but excluding the atom carrying the bond with the " 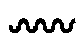 " symbol and the bridging carbon atoms.

When there are more than one such depicted "floating" groups, as for example in the formulae: 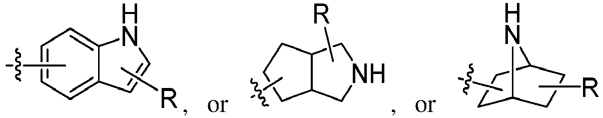 where there are two groups, namely, the R and the bond indicating attachment to a parent structure then, unless otherwise defined, each "floating" group can reside on any atoms of the ring system, again assuming each replaces a depicted, implied, or expressly defined hydrogen on the ring system and a chemically stable compound would be formed by such an arrangement.

When a group R is depicted as existing on a ring system containing saturated carbons, for example as in the formula: 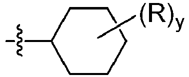 where, in this example, y can be more than one, and assuming each R replaces a currently depicted implied, or expressly defined hydrogen on the ring; then, unless otherwise defined, two R's can reside on the same carbon. A simple example is when R is a methyl group. The depicted structure can exist as a geminal dimethyl on a carbon of the depicted ring (an "annular" carbon). In another example, two R's on the same carbon, including that same carbon, can form a ring, thus creating a spirocyclic ring (a "spirocyclyl" group) structure. For example, shown below two Rs can form a piperidine ring in a spirocyclic arrangement with the cyclohexane, as

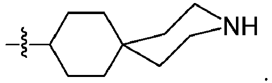

A person of ordinary skill in the art will appreciate that the definitions may be combined to further describe a particular compound. For example, hydroxyaliphatic refers to an aliphatic group substituted with an hydroxy (-OH) group, and haloalkylaryl refers to an aryl group substituted with an alkyl group, where the alkyl group too is substituted with a halogen, and where the point of attachment to the parent structure is via the aryl moiety since aryl is the base name of the substituent.

As used herein, the term **"substituted"** refers to all subsequent modifiers in a term, for example in the term "substituted arylC₁₋₈alkyl," substitution may occur on the "C₁₋₈alkyl" portion, the "aryl" portion or both portions of the arylC₁₋₈alkyl group. Also by way of example, alkyl includes substituted cycloalkyl groups.

"Substituted," when used to modify a specified group or moiety, means that at least one, and perhaps two or more, hydrogen atoms of the specified group or moiety is independently replaced with the same or different substituent groups as defined below. In a particular embodiment, a group, moiety or substituent may be substituted or unsubstituted, unless expressly defined as either "unsubstituted" or "substituted." Accordingly, any of the groups specified herein may be unsubstituted or substituted. In particular embodiments, the substituent may or may not be expressly defined as substituted, but is still contemplated to be optionally substituted. For example, an "alkyl" or a "pyrazolyl" moiety may be unsubstituted or substituted, but an "unsubstituted alkyl" or an "unsubstituted pyrazolyl" is not substituted.

"Substituents" or "substituent groups" for substituting for one or more hydrogen atoms on saturated carbon atoms in the specified group or moiety are, unless otherwise specified, -R⁶⁰, halo, =O, -OR⁷⁰, -SR⁷⁰, -N(R⁸⁰)₂, haloalkyl, perhaloalkyl, -CN, -NO₂, =N₂, -N₃, -SO₂R⁷⁰, -SO₃⁻M⁺, -SO₃R⁷⁰, -OSO₂R⁷⁰, -OSO₃⁻M⁺, -OSO₃R⁷⁰, -P(O)(O⁻)₂(M⁺)₂, -P(O)(O⁻)₂M²⁺, -P(O)(OR⁷⁰)O⁻M⁺, -P(O)(OR⁷⁰)₂, -C(O)R⁷⁰, -C(S)R⁷⁰, -C(NR⁷⁰)R⁷⁰, -CO₂⁻M⁺, -CO₂R⁷⁰, -C(S)OR⁷⁰, -C(O)N(R⁸⁰)₂, -C(NR⁷⁰)(R⁸⁰)₂, -OC(O)R⁷⁰, -OC(S)R⁷⁰, -OCO₂⁻M⁺, -OCO₂R⁷⁰, -OC(S)OR⁷⁰, -NR⁷⁰C(O)R⁷⁰, -NR⁷⁰C(S)R⁷⁰, -NR⁷⁰CO₂⁻M⁺, -NR⁷⁰CO₂R⁷⁰, -NR⁷⁰C(S)OR⁷⁰, -NR⁷⁰C(O)N(R⁸⁰)₂, -NR⁷⁰C(NR⁷⁰)R⁷⁰ or -NR⁷⁰C(NR⁷⁰)N(R⁸⁰)₂, where R⁶⁰ is C₁₋₁₀aliphatic, heteroaliphatic, or cycloaliphatic, typically, C₁₋₆aliphatic, more typically C₁₋₆alkyl, where R⁶⁰ optionally may be substituted; each R⁷⁰ is independently for each occurrence hydrogen or R⁶⁰; each R⁸⁰ is independently for each occurrence R⁷⁰ or alternatively, two R⁸⁰ groups, taken together with the nitrogen atom to which they are attached, form a 3- to 7-membered heterocycloaliphatic, which optionally includes from 1 to 4 of the same or different additional heteroatoms selected from O, N and S, of which N optionally has R⁷⁰ substitution, such as H or C₁-C₃alkyl substitution; and each M⁺ is a counter ion with a net single positive charge. Each M⁺ is independently for each occurrence, for example, an alkali metal ion, such as K⁺, Na⁺, Li⁺; an ammonium ion, such as ⁺N(R⁷⁰)₄; a protonated amino acid ion, such as a lysine ion, or an arginine ion; or an alkaline metal earth ion, such as [Ca²⁺]_{0.5}, [Mg²⁺]_{0.5}, or [Ba²⁺]_{0.5} (a subscript "0.5" means, for example, that one of the counter ions for such divalent alkali earth ions can be an ionized form of a compound of the invention and the other is a typical counter ion such as chloride, or two ionized compounds can serve as counter ions for such divalent alkali earth ions, or alternatively, a doubly ionized compound can serve as the counter ion for such divalent alkali earth ions). As specific examples, -N(R⁸⁰)₂ includes -NH₂, -NH-alkyl, -NH-pyrrolidin-3-yl, *N*-pyrrolidinyl, *N*-piperazinyl, 4*N*-methyl-piperazin-1-yl, *N*-morpholinyl and the like. Any two hydrogen atoms on a single carbon also can be replaced with, for example, =O, =NR⁷⁰, =N-OR⁷⁰, =N₂ or =S.

Substituent groups for replacing hydrogen atoms on unsaturated carbon atoms in groups containing unsaturated carbons are, unless otherwise specified, -R⁶⁰, halo, -O⁻M⁺, -OR⁷⁰, -SR⁷⁰, -S⁻ M⁺, -N(R⁸⁰)₂, perhaloalkyl, -CN, -OCN, -SCN, -NO, -NO₂, -N₃, -SO₂R⁷⁰, -SO3⁻M⁺, -SO₃R⁷⁰, -OSO₂R⁷⁰, -OSO₃⁻M⁺, -OSO₃R⁷⁰, -PO₃⁻²(M⁺)₂, -PO₃⁻²M²⁺, -P(O)(OR⁷⁰)O⁻M⁺, -P(O)(OR⁷⁰)₂, -C(O)R⁷⁰, -C(S)R⁷⁰, -C(NR⁷⁰)R⁷⁰, -CO₂⁻M⁺, -CO₂R⁷⁰, -C(S)OR⁷⁰, -C(O)NR⁸⁰R⁸⁰, -C(NR⁷⁰)N(R⁸⁰)₂, -OC(O)R⁷⁰, -OC(S)R⁷⁰, -OCO₂⁻M⁺, -OCO₂R⁷⁰, -OC(S)OR⁷⁰, -NR⁷⁰C(O)R⁷⁰, -NR⁷⁰C(S)R⁷⁰, -NR⁷⁰CO₂⁻M⁺, -NR⁷⁰CO₂R⁷⁰, -NR⁷⁰C(S)OR⁷⁰, -NR⁷⁰C(O)N(R⁸⁰)₂, -NR⁷⁰C(NR⁷⁰)R⁷⁰ or -NR⁷⁰C(NR⁷⁰)N(R⁸⁰)₂, where R⁶⁰, R⁷⁰, R⁸⁰ and M⁺ are as previously defined, provided that in case of substituted alkene or alkyne, the substituents are not -O⁻M⁺, -OR⁷⁰, -SR⁷⁰, or -S⁻M⁺.

Substituent groups for replacing hydrogen atoms on nitrogen atoms in groups containing such nitrogen atoms are, unless otherwise specified, -R⁶⁰, -O⁻M⁺, -OR⁷⁰, -SR⁷⁰, -S⁻M⁺, -N(R⁸⁰)₂, perhaloalkyl, -CN, -NO, -NO₂, -S(O)₂R⁷⁰, -SO₃⁻M⁺, -SO₃R⁷⁰, -OS(O)₂R⁷⁰, -OSO₃⁻M⁺, -OSO₃R⁷⁰, -PO₃²⁻(M⁺)₂, -PO₃²⁻M²⁺, -P(O)(OR⁷⁰)O⁻M⁺, -P(O)(OR⁷⁰)(OR⁷⁰), -C(O)R⁷⁰, -C(S)R⁷⁰, -C(NR⁷⁰)R⁷⁰, -CO₂R⁷⁰, -C(S)OR⁷⁰, -C(O)NR⁸⁰R⁸⁰, -C(NR⁷⁰)NR⁸⁰R⁸⁰, -OC(O)R⁷⁰, -OC(S)R⁷⁰, -OCO₂R⁷⁰, -OC(S)OR⁷⁰, -NR⁷⁰C(O)R⁷⁰, -NR⁷⁰C(S)R⁷⁰, -NR⁷⁰CO₂R⁷⁰, -NR⁷⁰C(S)OR⁷⁰, -NR⁷⁰C(O)N(R⁸⁰)₂, -NR⁷⁰C(NR⁷⁰)R⁷⁰ or -NR⁷⁰C(NR⁷⁰)N(R⁸⁰)₂, where R⁶⁰, R⁷⁰, R⁸⁰ and M⁺ are as previously defined.

In one embodiment, a group that is substituted has 1 substituent, 2 substituents, substituents, or 4 substituents.

Additionally, in embodiments where a group or moiety is substituted with a substituted substituent, the nesting of such substituted substituents is limited to three, thereby preventing the formation of polymers. Thus, in a group or moiety comprising a first group that is a substituent on a second group that is itself a substituent on a third group, which is attached to the parent structure, the first (outermost) group can only be substituted with unsubstituted substituents. For example, in a group comprising -(aryl-1)-(aryl-2)-(aryl-3), aryl-3 can only be substituted with substituents that are not themselves substituted.

**"Aliphatic"** refers to a substantially hydrocarbon-based group or moiety. An aliphatic group or moiety can be acyclic, including **alkyl, alkenyl,** or **alkynyl** groups, cyclic versions thereof, such as **cycloaliphatic** groups or moieties including **cycloalkyl, cycloalkenyl** or **cycloalkynyl,** and further including straight- and branched-chain arrangements, and all stereo and position isomers as well. Unless expressly stated otherwise, an aliphatic group contains from one to twenty-five carbon atoms (C₁₋₂₅); for example, from one to fifteen (C₁₋₁₅), from one to ten (C₁₋₁₀), from one to six (C₁₋₆), or from one to four carbon atoms (C₁₋₄) for a saturated acyclic aliphatic group or moiety, from two to twenty-five carbon atoms (C₂₋₂₅); for example, from two to fifteen (C₂₋₁₅), from two to ten (C₂₋₁₀), from two to six (C₂₋₆), or from two to four carbon atoms (C₂₋₄) for an unsaturated acyclic aliphatic group or moiety, or from three to fifteen (C₃₋₁₅) from three to ten (C₃₋₁₀), from three to six (C₃₋₆), or from three to four (C₃₋₄) carbon atoms for a cycloaliphatic group or moiety. An aliphatic group may be substituted or unsubstituted, unless expressly referred to as an "unsubstituted aliphatic" or a "substituted aliphatic." An aliphatic group can be substituted with one or more substituents (up to two substituents for each methylene carbon in an aliphatic chain, or up to one substituent for each carbon of a -C=C- double bond in an aliphatic chain, or up to one substituent for a carbon of a terminal methine group).

"Alkoxy" refers to the group -OR, where R is a substituted or unsubstituted alkyl or a substituted or unsubstituted cycloalkyl group. In certain examples R is a C₁₋₆ alkyl group or a C₃₋₆cycloalkyl group. Methoxy (-OCH₃) and ethoxy (-OCH₂CH₃) are exemplary alkoxy groups. In a substituted alkoxy, R is substituted alkyl or substituted cycloalkyl, examples of which include haloalkoxy groups, such as -OCF₂H, or -OCF₃.

"Alkyl" refers to a saturated aliphatic hydrocarbyl group having from 1 to 25 (C₁₋₂₅) or more carbon atoms, more typically 1 to 10 (C₁₋₁₀) carbon atoms such as 1 to 8 (C₁₋₈) carbon atoms, 1 to 6 (C₁₋₆) carbon atoms or 1 to 4 (C₁₋₄) carbon atoms. An alkyl moiety may be substituted or unsubstituted. This term includes, by way of example, linear and branched hydrocarbyl groups such as methyl (CH₃), ethyl (-CH₂CH₃), n-propyl (-CH₂CH₂CH₃), isopropyl (-CH(CH₃)₂), n-butyl (-CH₂CH₂CH₂CH₃), isobutyl (-CH₂CH₂(CH₃)₂), sec-butyl (-CH(CH₃)(CH₂CH₃), t-butyl (-C(CH₃)₃), n-pentyl (-CH₂CH₂CH₂CH₂CH₃), and neopentyl (-CH₂C(CH₃)₃). As used herein, "lower alkyl" means (C₁-C₈) alkyl.

**"Aromatic"** refers to a cyclic, conjugated group or moiety of, unless specified otherwise, from 5 to 15 ring atoms having a single ring (e.g., phenyl, pyridinyl, or pyrazolyl) or multiple condensed rings in which at least one ring is aromatic (e.g., naphthyl, indolyl, or pyrazolopyridinyl), that is at least one ring, and optionally multiple condensed rings, have a continuous, delocalized π-electron system. Typically, the number of out of plane π-electrons corresponds to the Hückel rule (4n + 2). The point of attachment to the parent structure typically is through an aromatic portion of the condensed ring system. For example, 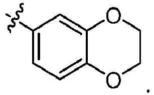 However, in certain examples, context or express disclosure may indicate that the point of attachment is through a non-aromatic portion of the condensed ring system. For example, 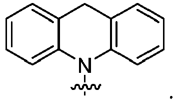 An aromatic group or moiety may comprise only carbon atoms in the ring, such as in an aryl group or moiety, or it may comprise one or more ring carbon atoms and one or more ring heteroatoms comprising a lone pair of electrons (e.g. S, O, N, P, or Si), such as in a heteroaryl group or moiety. Unless otherwise stated, an aromatic group may be substituted or unsubstituted.

**"Aryl"** refers to an aromatic carbocyclic group of, unless specified otherwise, from 6 to 15 carbon atoms having a single ring (e.g., phenyl) or multiple condensed rings in which at least one ring is aromatic multiple condensed rings in which at least one ring is aromatic (e.g., 1,2,3,4-tetrahydroquinoline, benzodioxole, and the like) providing that the point of attachment is through an aromatic portion of the ring system. If any aromatic ring portion contains a heteroatom, the group is heteroaryl and not aryl. Aryl groups may be, for example, monocyclic, bicyclic, tricyclic or tetracyclic. Unless otherwise stated, an aryl group may be substituted or unsubstituted.

**"Araliphatic"** refers to an aryl group attached to the parent via an aliphatic moiety. Araliphatic includes aralkyl or arylalkyl groups such as benzyl and phenylethyl.

**"Cyano"** refers to the group -CN.

**"Cycloaliphatic"** refers to a cyclic aliphatic group having a single ring (*e.g*., cyclohexyl), or multiple rings, such as in a fused, bridged or spirocyclic system, at least one of which is aliphatic. Typically, the point of attachment to the parent structure is through an aliphatic portion of the multiple ring system. Cycloaliphatic includes saturated and unsaturated systems, including **cycloalkyl, cycloalkenyl** and **cycloalkynyl.** A cycloaliphatic group may contain from three to twenty-five carbon atoms; for example, from three to fifteen, from three to ten, or from three to six carbon atoms. Unless otherwise stated, a cycloaliphatic group may be substituted or unsubstituted. Exemplary cycloaliphatic groups include, but are not limited to, cyclopropyl, cyclobutyl, cyclopentyl, cyclohexyl, cycloheptyl, cyclopentenyl, or cyclohexenyl. As used herein, lower cycloalkyl refers to C₃₋₈cycloalkyl.

**"Halo," "halide"** or **"halogen"** refers to fluoro, chloro, bromo or iodo.

**"Heteroaliphatic"** refers to an aliphatic compound or group having at least one heteroatom and at least one carbon atom, *i.e.,* one or more carbon atoms from an aliphatic compound or group comprising at least two carbon atoms, has been replaced with an atom having at least one lone pair of electrons, typically nitrogen, oxygen, phosphorus, silicon, or sulfur. For example, a **heteroalkyl** moiety is a heteroaliphatic moiety where the base aliphatic moiety is an alkyl as defined herein. Heteroaliphatic compounds or groups may be substituted or unsubstituted, branched or unbranched, chiral or achiral, and/or acyclic or cyclic, such as a heterocycloaliphatic group.

**"Heteroaryl"** refers to an aromatic group or moiety of, unless specified otherwise, from 5 to 15 ring atoms comprising at least one carbon atom and at least one heteroatom, such as N, S, O, P, or Si. A heteroaryl group or moiety may comprise a single ring (e.g., pyridinyl, pyrimidinyl or pyrazolyl) or multiple condensed rings (e.g., indolyl, benzopyrazolyl, or pyrazolopyridinyl). Heteroaryl groups or moiety may be, for example, monocyclic, bicyclic, tricyclic or tetracyclic. Unless otherwise stated, a heteroaryl group or moiety may be substituted or unsubstituted.

**"Heterocyclyl,"** "**heterocyclo**" and **"heterocycle"** refer to both aromatic and non-aromatic ring systems, and more specifically refer to a stable three- to fifteen-membered ring moiety comprising at least one carbon atom, and typically plural carbon atoms, and at least one, such as from one to five, heteroatoms. The heteroatom(s) may be nitrogen, phosphorus, oxygen, silicon or sulfur atom(s). The heterocyclyl moiety may be a monocyclic moiety, or may comprise multiple rings, such as in a bicyclic or tricyclic ring system, provided that at least one of the rings contains a heteroatom. Such a multiple ring moiety can include fused or bridged ring systems as well as spirocyclic systems; and any nitrogen, phosphorus, carbon, silicon or sulfur atoms in the heterocyclyl moiety can be optionally oxidized to various oxidation states. For convenience, nitrogens, particularly, but not exclusively, those defined as annular aromatic nitrogens, are meant to include their corresponding N-oxide form, although not explicitly defined as such in a particular example. Thus, for a compound having, for example, a pyridinyl ring, the corresponding pyridinyl-N-oxide is included as another compound of the invention, unless expressly excluded or excluded by context. In addition, annular nitrogen atoms can be optionally quaternized. Heterocycle includes **heteroaryl** moieties, where the heterocylyl moieties are aromatic, and **heterocycloaliphatic** moieties, such as heterocycloalkyl, heterocycloalkenyl, or heterocycloalkynyl, which are heterocyclyl rings that are partially or fully saturated. Examples of heterocyclyl groups include, but are not limited to, azetidinyl, oxetanyl, acridinyl, benzodioxolyl, benzodioxanyl, benzofuranyl, carbazoyl, cinnolinyl, dioxolanyl, indolizinyl, naphthyridinyl, perhydroazepinyl, phenazinyl, phenothiazinyl, phenoxazinyl, phthalazinyl, pteridinyl, purinyl, quinazolinyl, quinoxalinyl, quinolinyl, isoquinolinyl, tetrazoyl, tetrahydroisoquinolyl, piperidinyl, piperazinyl, 2-oxopiperazinyl, 2-oxopiperidinyl, 2-oxopyrrolidinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, dihydropyridinyl, tetrahydropyridinyl, pyridinyl, pyrazinyl, pyrimidinyl, pyridazinyl, oxazolyl, oxazolinyl, oxazolidinyl, triazolyl, isoxazolyl, isoxazolidinyl, morpholinyl, thiazolyl, thiazolinyl, thiazolidinyl, isothiazolyl, quinuclidinyl, isothiazolidinyl, indolyl, isoindolyl, indolinyl, isoindolinyl, octahydroindolyl, octahydroisoindolyl, quinolyl, isoquinolyl, decahydroisoquinolyl, benzimidazolyl, thiadiazolyl, benzopyranyl, benzothiazolyl, benzoxazolyl, furyl, diazabicycloheptane, diazapane, diazepine, tetrahydrofuryl, tetrahydropyranyl, thienyl, benzothieliyl, thiamorpholinyl, thiamorpholinyl sulfoxide, thiamorpholinyl sulfone, dioxaphospholanyl, and oxadiazolyl.

**"Hydroxyl"** refers to the group -OH.

**"Nitro"** refers to the group -NO₂.

**"Oxo"** refers to the group =O (double bond O).

**"Phosphate"** refers to the group -O-P(O)(OR')₂, where each -OR' independently is -OH; - O-aliphatic, such as -O-alkyl or -O-cycloalkyl; -O-aromatic, including both -O-aryl and -O-heteroaryl; -O-aralkyl; or
-OR' is -O⁻M⁺, where M⁺ is a counter ion with a single positive charge. Each M⁺ may be an alkali ion, such as K⁺, Na⁺, Li⁺; an ammonium ion, such as ⁺N(R")₄ where each R" independently is H, aliphatic, heterocyclyl or aryl; or an alkaline earth ion, such as [Ca²⁺]_{0.5}, [Mg²⁺]_{0.5}, or [Ba²⁺]_{0.5}. Phosphonooxyalkyl refers to the group -alkyl-phosphate, such as, for example, -CH₂OP(O)(OH)₂, or a salt thereof, such as -CH₂OP(O)(O⁻Na⁺)₂, and (((dialkoxyphosphoryl)oxy)alkyl) refers to the dialkyl ester of a phosphonooxyalkyl group, such as, for example, -CH₂OP(O)(O-tert-butyl)₂.

**"Patient"** or **"Subject"** refers to mammals and other animals, particularly humans. Thus, disclosed methods are applicable to both human therapy and veterinary applications.

**"Pharmaceutically acceptable excipient"** refers to a substance, other than the active ingredient, that is included in a formulation of the active ingredient. As used herein, an excipient may be incorporated within particles of a pharmaceutical composition, or it may be physically mixed with particles of a pharmaceutical composition. An excipient can be used, for example, to dilute an active agent and/or to modify properties of a pharmaceutical composition. Excipients can include, but are not limited to, antiadherents, binders, coatings, enteric coatings, disintegrants, flavorings, sweeteners, colorants, lubricants, glidants, sorbents, preservatives, adjuvants, carriers or vehicles. Excipients may be starches and modified starches, cellulose and cellulose derivatives, saccharides and their derivatives such as disaccharides, polysaccharides and sugar alcohols, protein, synthetic polymers, crosslinked polymers, antioxidants, amino acids or preservatives. Exemplary excipients include, but are not limited to, magnesium stearate, stearic acid, vegetable stearin, sucrose, lactose, starches, hydroxypropyl cellulose, hydroxypropyl methylcellulose, xylitol, sorbitol, maltitol, gelatin, polyvinylpyrrolidone (PVP), polyethyleneglycol (PEG), tocopheryl polyethylene glycol 1000 succinate (also known as vitamin E TPGS, or TPGS), carboxy methyl cellulose, dipalmitoyl phosphatidyl choline (DPPC), vitamin A, vitamin E, vitamin C, retinyl palmitate, selenium, cysteine, methionine, citric acid, sodium citrate, methyl paraben, propyl paraben, sugar, silica, talc, magnesium carbonate, sodium starch glycolate, tartrazine, aspartame, benzalkonium chloride, sesame oil, propyl gallate, sodium metabisulphite or lanolin.

An **"adjuvant"** is an excipient that modifies the effect of other agents, typically the active ingredient. Adjuvants are often pharmacological and/or immunological agents. An adjuvant may modify the effect of an active ingredient by increasing an immune response. An adjuvant may also act as a stabilizing agent for a formulation. Exemplary adjuvants include, but are not limited to, aluminum hydroxide, alum, aluminum phosphate, killed bacteria, squalene, detergents, cytokines, paraffin oil, and combination adjuvants, such as freund's complete adjuvant or freund's incomplete adjuvant.

**"Pharmaceutically acceptable carrier"** refers to an excipient that is a carrier or vehicle, such as a suspension aid, solubilizing aid, or aerosolization aid. Pharmaceutically acceptable carriers are conventional. Remington: The Science and Practice of Pharmacy, The University of the Sciences in Philadelphia, Editor, Lippincott, Williams, & Wilkins, Philadelphia, PA, 21st Edition (2005), describes compositions and formulations suitable for pharmaceutical delivery of one or more therapeutic compositions and additional pharmaceutical agents.

In general, the nature of the carrier will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. In some examples, the pharmaceutically acceptable carrier may be sterile to be suitable for administration to a subject (for example, by parenteral, intramuscular, or subcutaneous injection). In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

**"Pharmaceutically acceptable salt"** refers to pharmaceutically acceptable salts of a compound that are derived from a variety of organic and inorganic counter ions as will be known to a person of ordinary skill in the art and include, by way of example only, sodium, potassium, calcium, magnesium, ammonium, tetraalkylammonium, and the like; and when the molecule contains a basic functionality, salts of organic or inorganic acids, such as hydrochloride, hydrobromide, tartrate, mesylate, acetate, maleate, oxalate, and the like. "Pharmaceutically acceptable acid addition salts" are a subset of "pharmaceutically acceptable salts" that retain the biological effectiveness of the free bases while formed by acid partners. In particular, the disclosed compounds form salts with a variety of pharmaceutically acceptable acids, including, without limitation, inorganic acids such as hydrochloric acid, hydrobromic acid, hydroiodic acid, sulfuric acid, nitric acid, phosphoric acid, and the like, as well as organic acids such as formic acid, acetic acid, trifluoroacetic acid, propionic acid, glycolic acid, pyruvic acid, oxalic acid, malic acid, maleic acid, malonic acid, succinic acid, fumaric acid, tartaric acid, citric acid, benzoic acid, 3-(4-hydroxybenzoyl) benzoic acid, cinnamic acid, mandelic acid, benzene sulfonic acid, isethionic acid, salicylic acid, xinafoic acid, lactic acid, palmitic acid, alkylsulfonic acids (for example, methanesulfonic acid, ethanesulfonic acid, 1,2-ethane-disulfonic acid, 2-hydroxyethanesulfonic acid, etc.), arylsulfonic acids (for example, benzenesulfonic acid, 4-chlorobenzenesulfonic acid, 2-naphthalenesulfonic acid, 4-toluenesulfonic acid, camphorsulfonic acid, etc.), 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like. Pharmaceutically acceptable salts also include salts formed when an acidic proton present in the parent compound is either replaced by a metal ion (for example, an alkali metal ion, an alkaline earth metal ion or an aluminum ion) or coordinates with an organic base (for example, ethanolamine, diethanolamine, triethanolamine, N-methylglucamine, morpholine, piperidine, dimethylamine, diethylamine, triethylamine, ammonia, etc.).

"Pharmaceutically acceptable base addition salts" are a subset of "pharmaceutically acceptable salts" that are derived from inorganic bases such as sodium, potassium, lithium, ammonium, calcium, magnesium, iron, zinc, copper, manganese, aluminum salts and the like. Exemplary salts are the ammonium, potassium, sodium, calcium, and magnesium salts. Salts derived from pharmaceutically acceptable organic bases include, but are not limited to, salts of primary, secondary, and tertiary amines, substituted amines including naturally occurring substituted amines, cyclic amines and basic ion exchange resins, such as isopropylamine, trimethylamine, diethylamine, triethylamine, tripropylamine, tris(hydroxymethyl)aminomethane (Tris), ethanolamine, 2-dimethylaminoethanol, 2-diethylaminoethanol, dicyclohexylamine, lysine, arginine, histidine, caffeine, procaine, hydrabamine, choline, betaine, ethylenediamine, glucosamine, methylglucamine, theobromine, purine, piperazine, piperidine, N-ethylpiperidine, polyamine resins, and the like. Exemplary organic bases are isopropylamine, diethylamine, tris(hydroxymethyl)aminomethane (Tris), ethanolamine, trimethylamine, dicyclohexylamine, choline, and caffeine. (See, for example, S. M. Berge, et al., "Pharmaceutical Salts," J. Pharm. Sci., 1977; 66:1-19.)

**"Effective amount,"** such as a therapeutically effective amount, refer to an amount of a compound sufficient to achieve a desired result, for example, to treat a specified disorder or disease, or to ameliorate or eradicate one or more of its symptoms and/or to prevent the occurrence of the disease or disorder. The amount of a compound which constitutes an "effective amount" will vary depending on the compound, the disease state and its severity, the age of the patient to be treated, and the like. The effective amount can be determined by a person of ordinary skill in the art. An appropriate "effective" amount in any individual case can be determined using any suitable technique, such as a dose escalation study.

**"Prodrug"** refers to compounds that are transformed *in vivo* to yield a biologically active compound, particularly the parent compound, for example, by hydrolysis in the gut or enzymatic conversion. Common examples of prodrug moieties include, but are not limited to, ester and amide forms of a compound having an active form bearing a carboxylic acid moiety. Examples of pharmaceutically acceptable esters suitable for use with the disclosed compounds include, but are not limited to, esters of phosphate groups and carboxylic acids, such as aliphatic esters, particularly alkyl esters (for example C₁₋₆alkyl esters). Other prodrug moieties include phosphate esters, such as -CH₂-O-P(O)(OR')₂ or a salt thereof, wherein R' is H or C₁₋₆alkyl. Acceptable esters also include cycloalkyl esters and arylalkyl esters such as, but not limited to benzyl. Examples of pharmaceutically acceptable amides of the disclosed compounds include, but are not limited to, primary amides, and secondary and tertiary alkyl amides (for example with between about one and about six carbons). Amides and esters of the disclosed compounds can be prepared according to conventional methods. A thorough discussion of prodrugs is provided in T. Higuchi and V. Stella, "Pro-drugs as Novel Delivery Systems," Vol 14 of the A.C.S. Symposium Series, and in Bioreversible Carriers in Drug Design, ed. Edward B. Roche, American Pharmaceutical Association and Pergamon Press, 1987.

**"Protecting group"** refers to a group of atoms that, when attached to a reactive functional group in a molecule, mask, reduce or prevent the reactivity of the functional group. Typically, a protecting group may be selectively removed as desired during the course of a synthesis. Examples of protecting groups can be found in Greene and Wuts, Protective Groups in Organic Chemistry, 3rd Ed., 1999, John Wiley & Sons, NY and Harrison et al., Compendium of Synthetic Organic Methods, Vols. 1-8, 1971-1996, John Wiley & Sons, NY. Representative amino protecting groups include, but are not limited to, formyl, acetyl, trifluoroacetyl, benzyl, benzyloxycarbonyl ("CBZ"), tert-butoxycarbonyl ("Boc"), trimethylsilyl ("TMS"), 2-trimethylsilyl-ethanesulfonyl ("TES"), trityl and substituted trityl groups, allyloxycarbonyl, 9-fluorenylmethyloxycarbonyl ("FMOC"), nitro-veratryloxycarbonyl ("NVOC") and the like. Representative hydroxyl protecting groups include, but are not limited to, those where the hydroxyl group is either acylated or alkylated such as benzyl and trityl ethers, as well as alkyl ethers, tetrahydropyranyl ethers, trialkylsilyl ethers (*e.g.,* TMS or TIPPS groups) and allyl ethers.

**"Solvate"** refers to a complex formed by combination of solvent molecules with molecules or ions of the solute. The solvent can be an organic compound, an inorganic compound, or a mixture of both. Some examples of solvents include, but are not limited to, methanol, ethanol, isopropanol, ethyl acetate, N,N-dimethylformamide, tetrahydrofuran, dimethylsulfoxide, and water. The compounds described herein can exist in un-solvated as well as solvated forms when combined with solvents, pharmaceutically acceptable or not, such as water, ethanol, and the like. Solvated and unsolvated forms of the presently disclosed compounds are within the scope of the embodiments disclosed herein.

**"Treating"** or **"treatment"** as used herein concerns treatment of CRS in a patient or subject, particularly a human experiencing CRS, and includes by way of example, and without limitation:
(i) inhibiting CRS, for example, arresting or slowing its development;
(ii) relieving CRS, for example, causing regression of CRS or a symptom thereof; or
(iii) stabilizing CRS, such as by preventing the CRS from increasing in grade and/or severity.

**"Preventing"** as used herein concerns preventing CRS from occurring in a patient or subject, in particular, when such patient or subject is at risk of developing CRS but has not yet been diagnosed as having it.

As used herein, the terms "disease" and "condition" can be used interchangeably or can be different in that the particular malady or condition may not have a known causative agent (so that etiology has not yet been determined) and it is therefore not yet recognized as a disease but only as an undesirable condition or syndrome, where a more or less specific set of symptoms have been identified by clinicians.

The above definitions and the following general formulas are not intended to include impermissible substitution patterns (e.g., methyl substituted with 5 fluoro groups). Such impermissible substitution patterns are easily recognized by a person having ordinary skill in the art.

Any of the groups referred to herein may be optionally substituted by at least one, possibly two or more, substituents as defined herein. That is, a substituted group has at least one, possible two or more, substitutable hydrogens replaced by a substituent or substituents as defined herein, unless the context indicates otherwise or a particular structural formula precludes substitution.

A person of ordinary skill in the art will appreciate that compounds may exhibit the phenomena of tautomerism, conformational isomerism, geometric isomerism, and/or optical isomerism. For example, certain disclosed compounds can include one or more chiral centers and/or double bonds and as a consequence can exist as stereoisomers, such as double-bond isomers (i.e., geometric isomers), enantiomers, diasteromers, and mixtures thereof, such as racemic mixtures. Accordingly, compounds and compositions may be provided as individual pure enantiomers or diasteriomers, or as stereoisomeric mixtures, including racemic mixtures. In certain embodiments the compounds disclosed herein are synthesized in or are purified to be in substantially enantiopure form, such as in an 85% enantiomeric excess (e.e.), a 90% enantiomeric excess, a 95% enantiomeric excess, a 97% enantiomeric excess, a 98% enantiomeric excess, a 99% enantiomeric excess, or even in greater than a 99% enantiomeric excess, such as in a substantially enantiopure form. A person of ordinary skill in the art understands that in a compound comprising one or more asymmetric centers, one or both enantiomers or diastereomers are contemplated unless a specific enantiomer or diastereomer is shown or described.

As another example, certain disclosed compounds can exist in several tautomeric forms, including the enol form, the keto form, and mixtures thereof. As the various compound names, formulae and compound drawings within the specification and claims can represent only one of the possible tautomeric, conformational isomeric, optical isomeric, or geometric isomeric forms, a person of ordinary skill in the art will appreciate that the disclosed compounds encompass any tautomeric, conformational isomeric, optical isomeric, and/or geometric isomeric forms of the compounds described herein, as well as mixtures of these various different isomeric forms. In cases of limited rotation, e.g. around the amide bond or between two directly attached rings such as the pyrazolyl and pyridinyl rings, atropisomers are also possible and are also specifically included in the compounds of the invention.

In any embodiments, any or all hydrogens present in the compound, or in a particular group or moiety within the compound, may be replaced by a deuterium or a tritium. Thus, a recitation of alkyl includes deuterated alkyl, where from one to the maximum number of hydrogens present may be replaced by deuterium. For example, ethyl may be C₂H₅ or C₂H₅ where from 1 to 5 hydrogens are replaced by deuterium, such as in C₂DₓH₅₋ₓ.

### II. Compounds

Disclosed herein are compounds, prodrugs, corresponding salt and/or solvate forms, and methods of using these compounds, prodrugs, and salt/solvate forms for treating and/or preventing CRS. The compounds may be compounds that modulate spleen tyrosine kinase (Syk) and/or may be a kinase inhibitor, such as a Syk inhibitor. The compound is a pyrimidine diamine according to formula I 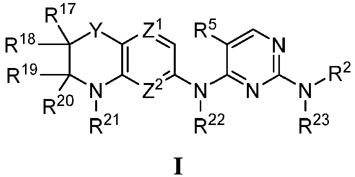 or a salt, solvate, N-oxide or prodrug thereof. In some embodiments, compounds of Formula I are Syk inhibitors. With respect to Formula I, Y is selected from CH₂, NR²⁴, O, S, S(O) and S(O)₂. In some embodiments, Y is O.

Z¹ and Z² are each, independently of one another, selected from CH and N. In some embodiments, Z¹ is CH. In some embodiments, Z² is N. In certain embodiments, Z¹ is CH and Z² is N.

R² is selected from (C₁-C₈) alkyl optionally substituted with one or more of the same or different R⁸ groups, (C₃-C₈) cycloalkyl optionally substituted with one or more of the same or different R⁸ groups, cyclohexyl optionally substituted with one or more of the same or different R⁸ groups, 3-8 membered heterocycloalkyl optionally substituted with one or more of the same or different R⁸ groups, (C₆-C₁₄) aryl optionally substituted with one or more of the same or different R⁸ groups, phenyl optionally substituted with one or more of the same or different R⁸ groups and 5-15 membered heteroaryl optionally substituted with one or more of the same or different R⁸ groups. In some embodiments, R² is phenyl optionally substituted with one or more of the same or different R⁸ groups.

R⁵ is selected from halo, cyano, nitro, or trihalomethyl, such as trifluoromethyl. In some embodiments, R⁵ is halo, and may be F.

Each R⁸ independently is selected from R^{a}, R^{b}, R^{a} substituted with one or more, for example, from one to four, of the same or different R^{a} or R^{b}, -OR^{a} substituted with one or more of the same or different R^{a} or R^{b}, -B(OR^{a})₂, -B(NR^{c}R^{c})₂, -(CH₂)*ₘ*-R^{b}, -(CHR^{a})*ₘ*-R^{b}, -O-(CH₂)*ₘ*-R^{b}, -S-(CH₂)*ₘ*-R^{b}, -O-CHR^{a}R^{b}, -O-CR^{a}(R^{b})₂, -O-(CHR^{a})*ₘ*-R^{b}, -O- (CH₂)*ₘ*-CH[(CH₂)*ₘ*R^{b}]R^{b}, -S-(CHR^{a})*ₘ*-R^{b}, -C(O)NH-(CH₂)*ₘ*-R^{b}, -C(O)NH-(CHR^{a})*ₘ*-R^{b}, -O-(CH₂)*ₘ*-C(O)NH-(CH₂)*ₘ*-R^{b}, -S-(CH₂)*ₘ*-C(O)NH-(CH₂)*ₘ*-R^{b},-O-(CHR^{a})*ₘ*-C(O)NH-(CHR^{a})*ₘ*-R^{b}, -S-(CHR^{a})*ₘ*-C(O)NH-(CHR^{a})*ₘ*-R^{b}, -NH-(CH₂)*ₘ*-R^{b}, -NH-(CHR^{a})*ₘ*-R^{b}, -NH[(CH₂)*ₘ*R^{b}], -N[(CH₂)*ₘ*R^{b}]₂, -NH-C(O)-NH-(CH₂)*ₘ*-R^{b}, -NH-C(O)-(CH₂)*ₘ*-CHR^{b}R^{b} and -NH-(CH₂)*ₘ*-C(O)-NH-(CH₂)*ₘ*-R^{b}. In some embodiments, at least one, such as 1, 2, 3, 4 or more R⁸ is alkoxy and may be -O-(C₁₋₆)alkyl, such as methoxy.

R¹⁷ is selected from hydrogen, halogen, fluoro, (C₁-C₈) alkyl and methyl or, alternatively, R¹⁷ may be taken together with R¹⁸ to form an oxo (=O) group or, together with the carbon atom to which they are attached, a spirocycle containing from 3 to 7 carbon atoms. In some embodiments, R¹⁷ is C₁₋₆alkyl, such as methyl.

R¹⁸ is selected from hydrogen, halogen, fluoro, (C₁-C₈) alkyl and methyl or, alternatively, R¹⁸ may be taken together with R¹⁷ to form an oxo (=O) group or, together with the carbon atom to which they are attached, a spirocycle containing from 3 to 7 carbon atoms. In some embodiments, R¹⁸ is C₁₋₆alkyl, such as methyl.

R¹⁹ is selected from hydrogen, (C₁-C₈) alkyl, and methyl or, alternatively, R¹⁹ may be taken together with R²⁰ to form an oxo (=O) group or, together with the carbon atom to which they are attached, a spirocycle containing from 3 to 7 carbon atoms.

R²⁰ is selected from hydrogen, (C₁-C₈) alkyl and methyl or, alternatively, R²⁰ may be taken together with R¹⁹ to form an oxo (=O) group or, together with the carbon atom to which they are attached, a spirocycle containing from 3 to 7 carbon atoms. In some embodiments, R¹⁹ and R²⁰ together for an oxo group.

Each R^{a} is, independently of the others, selected from hydrogen, (C₁-C₈) alkyl, lower cycloalkyl, cyclohexyl, (C₄-C₁₁) cycloalkylalkyl, (C₆-C₁₀) aryl, phenyl, (C₇-C₁₆) arylalkyl, benzyl, 2-6 membered heteroalkyl, 3-8 membered heterocycloalkyl, morpholinyl, piperazinyl, homopiperazinyl, piperidinyl, 4-11 membered heterocycloalkylalkyl, 5-10 membered heteroaryl and 6-16 membered heteroarylalkyl.

Each R^{b} is a suitable group independently selected from =O, -OR^{a}, (C₁-C₃) haloalkyloxy, =S, -SR^{a}, =NR^{a}, =NOR^{a}, -NR^{c}R^{c}, halogen, -CF₃, -CN, -NC, -OCN, -SCN, -NO, -NO₂, =N₂, -N₃, -S(O)R^{a}, -S(O)₂R^{a}, -S(O)₂OR^{a}, -S(O)NR^{c}R^{c}, -S(O)₂NR^{c}R^{c}, -OS(O)R^{a}, -OS(O)₂R^{a}, -OS(O)₂OR^{a}, -OS(O)₂NR^{c}R^{c}, -C(O)R^{a}, -C(O)OR^{a}, -C(O)NR^{c}R^{c}, -C(NH)NR^{c}R^{c}, -C(NR^{a})NR^{c}R^{c}, -C(NOH)R^{a}, -C(NOH)NR^{c}R^{c}, -OC(O)R^{a}, -OC(O)OR^{a}, -OC(O)NR^{c}R^{c}, -OC(NH)NR^{c}R^{c}, -OC(NR^{a})NR^{c}R^{c}, -[NHC(O)]*ₙ*R^{a}, -[NR^{a}C(O)]*ₙ*R^{a}, -[NHC(O)]*ₙ*OR^{a}, -[NR^{a}C(O)]*ₙ*OR^{a}, -[NHC(O)]*ₙ*NR^{c}R^{c}, -[NR^{a}C(O)]*ₙ*NR^{c}R^{c}, -[NHC(NH)]*ₙ*NR^{c}R^{c} and -[NR^{a}C(NR^{a})]*ₙ*NR^{c}R^{c}.

Each R^{c} is, independently of the others, selected from an amino-protecting group and R^{a}, or, alternatively, the two R^{c} bonded to the same nitrogen atom are taken together with that nitrogen atom to form a 5 to 8-membered heterocycloalkyl or heteroaryl which may optionally include one or more of the same or different additional heteroatoms and which may optionally be substituted with one or more, for example, from one to four, of the same or different R^{a} groups.

The amino-protecting group may be any protecting group suitable to act as a protecting group for an amine moiety. In some embodiments, the amino-protecting group is formyl, acetyl, trifluoroacetyl, benzyl, benzyloxycarbonyl ("CBZ"), tert-butoxycarbonyl ("Boc"), trimethylsilyl ("TMS"), 2-trimethylsilyl-ethanesulfonyl ("TES"), trityl and substituted trityl groups, allyloxycarbonyl, 9-fluorenylmethyloxycarbonyl ("FMOC"), nitro-veratryloxycarbonyl ("NVOC") and the like.

R²¹, R²² and R²³ are each, independently of one another, selected from hydrogen and phosphonooxyalkyl.

R²⁴ is selected from hydrogen, (C₁-C₈) alkyl and phosphonooxyalkyl.

Each *m* is, independently of the others, an integer from 1 to 3.

Each *n* is, independently of the others, an integer from 0 to 3.

In some embodiments, at least one of R²¹, R²², R²³ and R²⁴ is phosphonooxyalkyl, but in other embodiments, none of R²¹, R²², R²³ and R²⁴ is phosphonooxyalkyl. In certain embodiments, each of R²¹, R²², R²³ and R²⁴ is hydrogen. In other certain embodiments, R²¹ is phosphonooxyalkyl and each of R²², R²³ and R²⁴ is hydrogen.

In some embodiments, a compound according to formula I has a formula II, III or IV 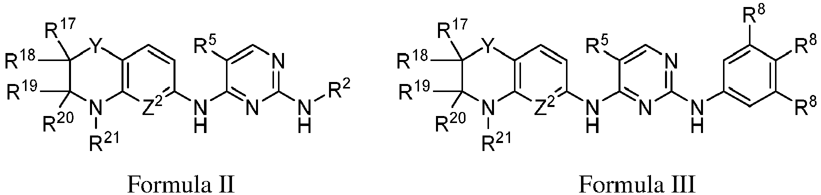 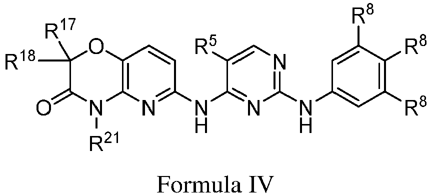 or a salt, solvate, N-oxide or prodrug thereof, where Y, Z², R², R⁵, R⁸, R¹⁷, R¹⁸, R¹⁹, R²⁰, and R²¹, if present, are as defined for formula I.

In some embodiments, the phosphonooxyalkyl moiety formulas I-IV is -CH₂OP(=O)(R³¹)₂, where each R³¹ independently is -OH, -O-aliphatic, such as -O-alkyl, or -O⁻M⁺, where M⁺ is a counter ion with a single positive charge.

In some embodiments, the compound has a formula V 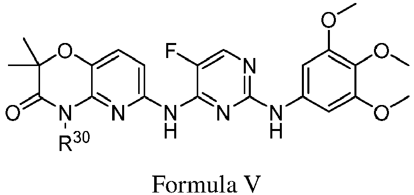 or a salt, solvate, N-oxide or prodrug thereof, where R³⁰ is H or phosphonooxyalkyl. In some embodiments, R³⁰ is H, but in other embodiments, R³⁰ is -CH₂OP(=O)(R³¹)₂, where each R³¹ independently is -OH, -O-aliphatic, such as -O-alkyl, or -O⁻M⁺, where M⁺ is a counter ion with a single positive charge.

Exemplary compounds according to one or more of formulas I-XI include: 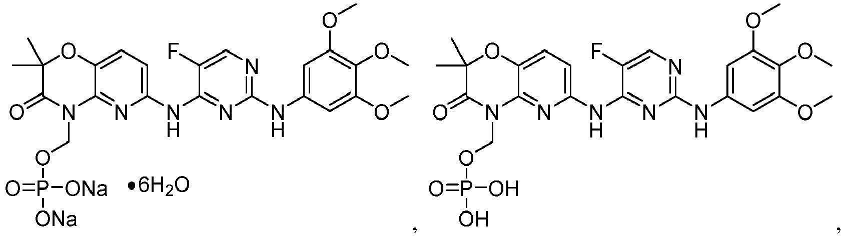 and

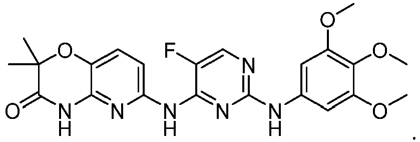

The FDA has approved TAVALISSE^{®} for the treatment of thrombocytopenia in adult patients with immune thrombocytopenia (ITP) who have had an insufficient response to a previous treatment. The active agent in TAVALISSE^{®} is fostamatinib disodium hexahydrate, [6-[[5-fluoro-2-(3,4,5-trimethoxyanilino)pyrimidin-4-yl]amino]-2,2-dimethyl-3-oxopyrido[3,2-b][1,4]oxazin-4-yl]methyl phosphate disodium hexahydrate, 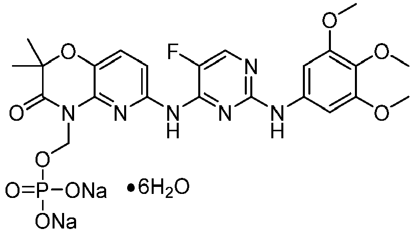 the disodium hexahydrate of fostamatinib (R788), [6-({5-Fluoro-2-[(3,4,5-trimethoxyphenyl)amino]-4-pyrimidinyl}amino)-2,2-dimethyl-3-oxo-2,3-dihydro-4*H*-pyrido[3,2-b][1,4]oxazin-4-yl]methyl dihydrogen phosphate,

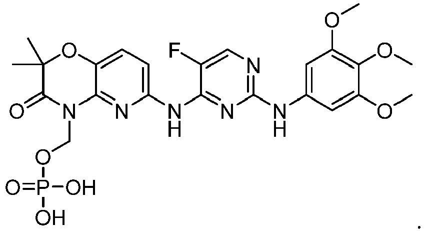

Fostamatinib and its disodium hexahydrate salt are prodrugs of the active metabolite 6-((5-fluoro-2-((3,4,5-trimethoxyphenyl)amino)pyrimidin-4-yl)amino)-2,2-dimethyl-2H-pyrido[3,2-b][1,4]oxazin-3(4H)-one (R406),

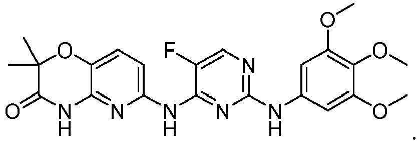

R406 is an inhibitor of the enzyme spleen tyrosine kinase (Syk), which plays a key role in the signaling of activating Fc receptors and the B-cell receptor (BCR). Syk is involved in the signal transduction pathways associated with the high affinity Fc receptors for IgE ("FcεRI") and/or IgG ("FcγRI") (*see* Valent et al., 2002, Intl. J. Hematol. 75(4):257-362 for review). Biochemical data confirm that 2,4-pyrimidinediamine compounds such as R406 exert degranulation inhibitory effect, at least in part, by blocking or inhibiting the signal transduction cascade(s) initiated by crosslinking of FcεRI and/or FcyRI (see, e.g., U.S. application Ser. No. 10/631,029 filed Jul. 29, 2003 (US2007/0060603, now U.S. Pat. No. 7,517,886), international application Ser. No. PCT/US03/24087 (WO2004/014382), U.S. application Ser. No. 10/903,263 filed Jul. 30, 2004 (US2005/0234049), and international application Ser. No. PCT/US2004/24716 (WO/2005/016893).

Braselmann et al. (J. Pharmacol. Exp. Ther. 319(3): 998-1008 (Dec. 2006; epub Aug. 31, 2006) demonstrated that R406 is a potent inhibitor of IgE- and IgG-mediated activation of Fc receptor signaling (EC₅₀ for degranulation = 56-64 nM). R406 inhibited phosphorylation of Syk substrate linker for activation of T cells in mast cells and B-cell linker protein/SLP65 in B cells. R406 bound to the ATP binding pocket of Syk and inhibited its kinase activity as an ATP-competitive inhibitor (K(i) = 30 nM). Furthermore, R406 blocked Syk-dependent FcR-mediated activation of monocytes/macrophages and neutrophils and BCR-mediated activation of B lymphocytes.

As used herein, "a form of fostamatinib" means fostamatinib, a pharmaceutically acceptable salt thereof, a pharmaceutically acceptable salt hydrate thereof, or a Syk-inhibiting metabolite thereof. In one embodiment, the form of fostamatinib is fostamatinib disodium hexahydrate (e.g., TAVALISSE^{®}). In another embodiment, the form of fostamatinib is the metabolite R406.

Disclosed herein is a method of blocking or ameliorating therapy-induced CRS by administering a form of fostamatinib (e.g., fostamatinib or a therapeutically acceptable salt thereof, e.g., fostamatinib disodium hexahydrate, and R406). The inventors have surprisingly found that a form of fostamatinib can block or ameliorate CRS toxicity or therapeutic rescue treatment in combination with steroids to lessen toxicity. A form of fostamatinib advantageously lower toxicity arising from CRS while maintaining efficacy of the immunotherapy/administered cells.

Additional information concerning compounds according to Formulas I-X can be found in U.S. Patent Nos. 7,449,458 and 8,163,902.

### IV. Compositions comprising a compound disclosed herein

The disclosed compounds may be used alone or in combination, and/or in combination with, or adjunctive to, at least one second therapeutic agent, and further the compound(s), and the at least one second therapeutic if present, may be used in combination with any suitable additive useful for forming compositions for administration to a subject. Additives can be included in pharmaceutical compositions for a variety of purposes, such as to dilute a composition for delivery to a subject, to facilitate processing of the formulation, to provide advantageous material properties to the formulation, to facilitate dispersion from a delivery device, to stabilize the formulation (e.g., antioxidants or buffers), to provide a pleasant or palatable taste or consistency to the formulation, or the like. Typical additives include, by way of example and without limitation: pharmaceutically acceptable excipient, including carriers and/or adjuvants, such as mono-, di-, and polysaccharides, sugar alcohols and other polyols, such as, lactose, glucose, raffinose, melezitose, lactitol, maltitol, trehalose, sucrose, mannitol, starch, or combinations thereof; surfactants, such as sorbitols, diphosphatidyl choline, and lecithin; bulking agents; buffers, such as phosphate and citrate buffers; anti-adherents, such as magnesium stearate; binders, such as saccharides (including disaccharides, such as sucrose and lactose,), polysaccharides (such as starches, cellulose, microcrystalline cellulose, cellulose ethers (such as hydroxypropyl cellulose), gelatin, synthetic polymers (such as polyvinylpyrrolidone, polyalkylene gylcols); coatings (such as cellulose ethers, including hydroxypropylmethyl cellulose, shellac, corn protein zein, and gelatin); release aids (such as enteric coatings); disintegrants (such as crospovidone, crosslinked sodium carboxymethyl cellulose, and sodium starch glycolate); fillers (such as dibasic calcium phosphate, vegetable fats and oils, lactose, sucrose, glucose, mannitol, sorbitol, calcium carbonate, and magnesium stearate); flavors and sweeteners (such as mint, cherry, anise, peach, apricot or licorice, raspberry, and vanilla; lubricants (such as minerals, exemplified by talc or silica, fats, exemplified by vegetable stearin, magnesium stearate or stearic acid); preservatives (such as antioxidants exemplified by vitamin A, vitamin E, vitamin C, retinyl palmitate, and selenium, amino acids, exemplified by cysteine and methionine, citric acid and sodium citrate, parabens, exemplified by methyl paraben and propyl paraben); colorants; compression aids; emulsifying agents; encapsulation agents; gums; granulation agents; and combinations thereof.

### V. Combinations of Therapeutic Agents

The disclosed compounds may be used alone, in combination with another disclosed compound, and/or as an adjunct to, or in combination with, other established therapies. In another aspect, the compounds may be used in combination with other therapeutic agents useful for treating CRS, and/or other diseases or conditions. The compounds and/or other agents may be administered simultaneously, sequentially in any order, by the same route of administration, or by a different route.

In some embodiments, a second therapeutic agent is an analgesic, an antibiotic, an anticoagulant, an antibody, an anti-inflammatory agent, an immunosuppressant, a guanylate cyclase-C agonist, an intestinal secretagogue, an antiviral, anticancer, antifungal, or a combination thereof. In certain embodiments, the second therapeutic is an anti-inflammatory agent, an immunosuppressant and/or may be a steroid.

The anti-inflammatory agent may be a steroid, such as budesonide, or a nonsteroidal anti-inflammatory agent. In certain embodiments, the nonsteroidal anti-inflammatory agent is selected from aminosalicylates (e.g., sulfasalazine, mesalamine, olsalazine, and balsalazide), cyclooxygenase inhibitors (COX-2 inhibitors, such as rofecoxib, celecoxib), diclofenac, etodolac, famotidine, fenoprofen, flurbiprofen, ketoprofen, ketorolac, ibuprofen, indomethacin, meclofenamate, mefenamic acid, meloxicam, nambumetone, naproxen, oxaprozin, piroxicam, salsalate, sulindac, tolmetin, or a combination thereof.

In some embodiments, the immunosuppressant is mercaptopurine; a corticosteroid, such as dexamethasone, hydrocortisone, prednisone, methylprednisolone and prednisolone; an alkylating agent, such as cyclophosphamide; a calcineurin inhibitor, such as cyclosporine, sirolimus and tacrolimus; an inhibitor of inosine monophosphate dehydrogenase (IMPDH) such as mycophenolate, mycophenolate mofetil and azathioprine; and agents designed to suppress cellular immunity while leaving the recipient's humoral immunologic response intact, including various antibodies (for example, antilymphocyte globulin (ALG), antithymocyte globulin (ATG), monoclonal anti-T-cell antibodies (OKT3)) and irradiation; or a combination thereof. In one embodiment, the antibody is infliximab. Azathioprine is currently available from Salix Pharmaceuticals, Inc. under the brand name Azasan; mercaptopurine is currently available from Gate Pharmaceuticals, Inc. under the brand name Purinethol; prednisone and prednisolone are currently available from Roxane Laboratories, Inc.; Methyl prednisolone is currently available from Pfizer; sirolimus (rapamycin) is currently available from Wyeth-Ayerst under the brand name Rapamune; tacrolimus is currently available from Fujisawa under the brand name Prograf; cyclosporine is current available from Novartis under the brand name Sandimmune and Abbott under the brand name Gengraf; IMPDH inhibitors such as mycophenolate mofetil and mycophenolic acid are currently available from Roche under the brand name Cellcept and Novartis under the brand name Myfortic; azathioprine is currently available from Glaxo Smith Kline under the brand name Imuran; and antibodies are currently available from Ortho Biotech under the brand name Orthoclone, Novartis under the brand name Simulect (basiliximab) and Roche under the brand name Zenapax (daclizumab).

In certain embodiments, the second therapeutic is, or comprises, a steroid, such as a corticosteroid, including, but not limited to, glucocorticoids and/or mineralocorticoids. Steroids suitable for use in combination with the disclosed compounds include synthetic and non-synthetic glucocorticoids. Exemplary steroids, such as glucocorticoids, suitable for use in the disclosed methods include, but are not limited to, alclomethasones, algestones, beclomethasones (e.g. beclomethasone dipropionate), betamethasones (e.g. betamethasone 17-valerate, betamethasone sodium acetate, betamethasone sodium phosphate, betamethasone valerate), budesonides, clobetasols (e.g. clobetasol propionate), clobetasones, clocortolones (e.g. clocortolone pivalate), cloprednols, corticosterones, cortisones, cortivazols, deflazacorts, desonides, desoximethasones, dexamethasones (e.g. dexamethasone 21-phosphate, dexamethasone acetate, dexamethasone sodium phosphate), diflorasones (e.g. diflorasone diacetate), diflucortolones, difluprednates, enoxolones, fluazacorts, flucloronides, fludrocortisones (e.g., fludrocortisone acetate), flumethasones (e.g. flumethasone pivalate), flunisolides, fluocinolones (e.g. fluocinolone acetonide), fluocinonides, fluocortins, fluocortolones, fluorometholones (e.g. fluorometholone acetate), fluperolones (e.g., fluperolone acetate), fluprednidenes, fluprednisolones, flurandrenolides, fluticasones (e.g. fluticasone propionate), formocortals, halcinonides, halobetasols, halometasones, halopredones, hydrocortamates, hydrocortisones (e.g. hydrocortisone 21-butyrate, hydrocortisone aceponate, hydrocortisone acetate, hydrocortisone buteprate, hydrocortisone butyrate, hydrocortisone cypionate, hydrocortisone hemisuccinate, hydrocortisone probutate, hydrocortisone sodium phosphate, hydrocortisone sodium succinate, hydrocortisone valerate), loteprednol etabonate, mazipredones, medrysones, meprednisones, methylprednisolones (methylprednisolone aceponate, methylprednisolone acetate, methylprednisolone hemi succinate, methylprednisolone sodium succinate), mometasones (e.g., mometasone furoate), paramethasones (e.g., paramethasone acetate), prednicarbates, prednisolones (e.g. prednisolone 25-diethylaminoacetate, prednisolone sodium phosphate, prednisolone 21-hemi succinate, prednisolone acetate; prednisolone famesylate, prednisolone hemisuccinate, prednisolone-21 (beta-D-glucuronide), prednisolone metasulphobenzoate, prednisolone steaglate, prednisolone tebutate, prednisolone tetrahydrophthalate), prednisones, prednivals, prednylidenes, rimexolones, tixocortols, triamcinolones (e.g. triamcinolone acetonide, triamcinolone benetonide, triamcinolone hexacetonide, triamcinolone acetonide 21-palmitate, triamcinolone diacetate), or any combination thereof. Additional information concerning steroids, and the salts thereof, can be found, for example, in Remington's Pharmaceutical Sciences, A. Osol, ed., Mack Pub. Co., Easton, Pa. (16th ed. 1980).

In some examples, the steroid is a glucocorticoid, and may be selected from cortisone, dexamethasone, hydrocortisone, methylprednisolone, prednisolone, prednisone, or a combination thereof. In a particular example, the steroid is, or comprises, prednisone. In another particular example, the steroid is, or comprises, dexamethasone.

### VI. Formulations and Administration

Pharmaceutical compositions comprising one or more of the disclosed compounds (including salts, solvates, N-oxides and/or prodrugs thereof) may be manufactured by means of conventional mixing, dissolving, granulating, dragee-making, levigating, emulsifying, encapsulating, entrapping or lyophilization processes. The compositions may be formulated in conventional manner using one or more physiologically acceptable excipients, diluents, carriers, adjuvants or auxiliaries to provide preparations which can be used pharmaceutically. A wide variety of suitable pharmaceutical compositions are known in the art. *See,* e.g., Remington: The Science and Practice of Pharmacy, volume I and volume II. (22nd Ed., University of the Sciences, Philadelphia).

The disclosed compound(s), or a prodrug thereof, may be formulated in the pharmaceutical compositions *per se,* or in the form of a solvate, N-oxide or pharmaceutically acceptable salt. Typically, such salts are more soluble in aqueous solutions than the corresponding free acids and bases, but salts having lower solubility than the corresponding free acids and bases may also be formed.

Pharmaceutical compositions comprising one or more of the disclosed compounds may take a form suitable for virtually any mode of administration, including, for example, topical, ocular, oral, buccal, systemic, nasal, injection, such as i.v. or i.p., transdermal, rectal, vaginal, sublingual, urethral (e.g., urethral suppository) etc., or a form suitable for administration by inhalation or insufflation. In certain embodiments, the mode of administration is oral or injection.

Systemic formulations include those designed for administration by injection, e.g., subcutaneous, intravenous, intramuscular, intrathecal or intraperitoneal injection, as well as those designed for transdermal, transmucosal oral or pulmonary administration.

Useful injectable preparations include sterile suspensions, solutions or emulsions of the active compound(s) in aqueous or oily vehicles. The compositions may also contain formulating agents, such as suspending, stabilizing and/or dispersing agent. The formulations for injection may be presented in unit dosage form, e.g., in ampules or in multidose containers, and may contain added preservatives.

Alternatively, the injectable formulation may be provided in powder form for reconstitution with a suitable vehicle, including but not limited to sterile, pyrogen-free water, buffer, dextrose solution, etc., before use. To this end, the disclosed compound(s) maybe dried by any art-known technique, such as lyophilization, and reconstituted prior to use.

For transmucosal administration, penetrants appropriate to the barrier to be permeated are used in the formulation. Such penetrants are known in the art.

For oral administration, the pharmaceutical compositions may take the form of, for example, lozenges, tablets or capsules prepared by conventional means with pharmaceutically acceptable excipients, such as: binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); and/or wetting agents (e.g., sodium lauryl sulfate). The tablets may be coated by methods well known in the art with, for example, sugars, films or enteric coatings.

Additionally, the pharmaceutical compositions containing the disclosed compound(s) as an active ingredient or solvates, N-oxides, pharmaceutically acceptable salts or prodrug(s) thereof in a form suitable for oral use, may also include, for example, troches, lozenges, aqueous or oily suspensions, dispersible powders or granules, emulsions, hard or soft capsules, or syrups or elixirs. Compositions intended for oral use can be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient (including a prodrug) in admixture with non-toxic pharmaceutically acceptable excipients which are suitable for the manufacture of tablets. These excipients can be for example, inert diluents, such as calcium carbonate, sodium carbonate, lactose, calcium phosphate or sodium phosphate; granulating and disintegrating agents (e.g., corn starch, or alginic acid); binding agents (e.g. starch, gelatin or acacia); and lubricating agents (e.g. magnesium stearate, stearic acid or talc). The tablets can be uncoated or they can be coated by known techniques to delay disintegration and absorption in the gastrointestinal tract and thereby provide a sustained action over a longer period. For example, a time delay material such as glyceryl monostearate or glyceryl distearate can be employed. They may also be coated by the techniques described in the U.S. Pat. Nos. 4,256,108; 4,166,452; and 4,265,874 to form osmotic therapeutic tablets for control release. The pharmaceutical compositions of the invention may also be in the form of oil-in-water emulsions. Tablets may also be film coated, and the file coating can comprise one or more of polyvinyl alcohol, titanium dioxide, polyethylene glycol 3350, talc, iron oxide yellow, and iron oxide red.

Liquid preparations for oral administration may take the form of, for example, elixirs, solutions, syrups or suspensions, or they may be presented as a dry product for constitution with water or other suitable vehicle before use. Such liquid preparations may be prepared by conventional means with pharmaceutically acceptable additives such as: suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol, cremophore^{™} or fractionated vegetable oils); and preservatives (e.g., methyl or propyl-p-hydroxybenzoates or sorbic acid). The preparations may also contain buffer salts, preservatives, flavoring, coloring and sweetening agents as appropriate.

Preparations for oral administration may be suitably formulated to give controlled release of the disclosed compound as is well known.

For buccal administration, the compositions may take the form of tablets or lozenges formulated in conventional manner.

For topical administration, the disclosed compound(s) (including solvates, N-oxides or pharmaceutically acceptable salt and/or prodrug(s) thereof) may be formulated as solutions, gels, ointments, creams, suspensions, etc. as are well-known in the art.

For rectal and vaginal routes of administration, the active compound(s) may be formulated as solutions (for retention enemas) suppositories or ointments containing conventional suppository bases, such as cocoa butter or other glycerides.

For nasal administration or administration by inhalation or insufflation, the disclosed compound(s), solvates, N-oxides, pharmaceutically acceptable salts or prodrug(s), can be conveniently delivered in the form of an aerosol spray from pressurized packs or a nebulizer with the use of a suitable propellant, e.g.,) dichlorodifluoromethane, trichlorofluoromethane, dichlorotetrafluoroethane, fluorocarbons, carbon dioxide or other suitable gas. In the case of a pressurized aerosol, the dosage unit may be determined by providing a valve to deliver a metered amount. Capsules and cartridges for use in an inhaler or insufflator (for example capsules and cartridges comprised of gelatin) may be formulated containing a powder mix of the compound and a suitable powder base such as lactose or starch.

The pharmaceutical compositions can be in the form of a sterile injectable aqueous or oleagenous suspension. This suspension can be formulated according to the known art using those suitable dispersing or wetting agents and suspending agents which have been mentioned above. The sterile injectable preparation may also be a sterile injectable solution or suspension in a non-toxic parenterally-acceptable diluent or solvent. Among the acceptable vehicles and solvents that can be employed are water, Ringer's solution and isotonic sodium chloride solution.

According to the present invention, a form of the disclosed compound(s), solvates, N-oxides, pharmaceutically acceptable salts or prodrug(s) thereof, can also be delivered by any of a variety of inhalation devices and methods known in the art, including, for example: U.S. Pat. No. 6,241,969; U.S. Pat. No. 6,060,069; U.S. Pat. No. 6,238,647; U.S. Pat. No 6,335,316; U.S. Pat. No. 5,364,838; U.S. Pat. No. 5,672,581; WO96/32149; WO95/24183; U.S. Pat. No. 5,654,007; U.S. Pat. No. 5,404,871; U.S. Pat. No. 5,672,581; U.S. Pat. No. 5,743,250; U.S. Pat. No. 5,419,315; U.S. Pat. No. 5,558,085; WO98/33480; U.S. Pat. No. 5,364,833; U.S. Pat. No. 5,320,094; U.S. Pat. No. 5,780,014; U.S. Pat. No. 5,658,878; 5,518,998; 5,506,203; U.S. Pat. No. 5,661,130; U.S. Pat. No. 5,655,523; U.S. Pat. No. 5,645,051; U.S. Pat. No. 5,622,166; U.S. Pat. No. 5,577,497; U.S. Pat. No. 5,492,112; U.S. Pat. No. 5,327,883; U.S. Pat. No. 5,277,195; U.S. Publication No. 20010041190; U.S. Publication No. 20020006901; and U.S. Publication No. 20020034477.

Included among the devices which can be used to administer a form of the active compound(s) are those well-known in the art, such as, metered dose inhalers, liquid nebulizers, dry powder inhalers, sprayers, thermal vaporizers, and the like. Other suitable technology for administration of particular 2,4-pyrimidinediamine compounds includes electrohydrodynamic aerosolizers.

In addition, the inhalation device is preferably practical, in the sense of being easy to use, small enough to carry conveniently, capable of providing multiple doses, and durable. Some specific examples of commercially available inhalation devices are Turbohaler (Astra, Wilmington, DE), Rotahaler (Glaxo, Research Triangle Park, NC), Diskus (Glaxo, Research Triangle Park, NC), the Ultravent nebulizer (Mallinckrodt), the Acorn II nebulizer (Marquest Medical Products, Totowa, NJ) the Ventolin metered dose inhaler (Glaxo, Research Triangle Park, NC), or the like. In one embodiment, the disclosed compound(s), solvates, N-oxides, pharmaceutically acceptable salts or prodrug(s) thereof can be delivered by a dry powder inhaler or a sprayer.

As those skilled in the art will recognize, the formulation of the form of the disclosed compound(s), solvates, N-oxides, pharmaceutically acceptable salts or prodrug(s) thereof, the quantity of the formulation delivered, and the duration of administration of a single dose depend on the type of inhalation device employed as well as other factors. For some aerosol delivery systems, such as nebulizers, the frequency of administration and length of time for which the system is activated will depend mainly on the concentration of the disclosed compound(s) in the aerosol. For example, shorter periods of administration can be used at higher concentrations the disclosed compound(s) in the nebulizer solution. Devices such as metered dose inhalers can produce higher aerosol concentrations, and can be operated for shorter periods to deliver the desired amount of active compound in some embodiments. Devices such as dry powder inhalers deliver active agent until a given charge of agent is expelled from the device. In this type of inhaler, the amount of the disclosed compound(s), solvates, N-oxides, pharmaceutically acceptable salts or prodrug(s) thereof in a given quantity of the powder determines the dose delivered in a single administration. The formulation of the disclosed compound(s) is selected to yield the desired particle size in the chosen inhalation device.

Formulations of a disclosed compound, such as a form of fostamatinib, for administration from a dry powder inhaler may typically include a finely divided dry powder containing the disclosed compound(s), but the powder can also include a bulking agent, buffer, carrier, excipient, another additive, or the like. Additives can be included in a dry powder formulation, for example, to dilute the powder as required for delivery from the particular powder inhaler, to facilitate processing of the formulation, to provide advantageous powder properties to the formulation, to facilitate dispersion of the powder from the inhalation device, to stabilize to the formulation (e.g., antioxidants or buffers), to provide taste to the formulation, or the like. Typical additives include mono-, di-, and polysaccharides; sugar alcohols and other polyols, such as, for example, lactose, glucose, raffinose, melezitose, lactitol, maltitol, trehalose, sucrose, mannitol, starch, or combinations thereof; surfactants, such as sorbitols, diphosphatidyl choline, or lecithin; or the like.

The method of the invention can be conducted a pharmaceutical composition including the disclosed compound(s), such as a form of fostamatinib, suitable for administration by inhalation. For example, a dry powder formulation can be manufactured in several ways, using conventional techniques, such as described in any of the publications mentioned above, and for example, Baker, et al., U.S. Pat. No. 5,700,904. Particles in the size range appropriate for maximal deposition in the lower respiratory tract can be made by micronizing, milling, or the like. And a liquid formulation can be manufactured by dissolving the compound in a suitable solvent, such as water, at an appropriate pH, including buffers or other excipients.

A specific example of an aqueous suspension formulation suitable for nasal administration using commercially-available nasal spray devices includes the following ingredients: active compound or prodrug (0.5 20 mg/ml); benzalkonium chloride (0.1 0.2 mg/mL); polysorbate 80 (TWEEN^{®} 80; 0.5 5 mg/ml); carboxymethylcellulose sodium or microcrystalline cellulose (1 15 mg/ml); phenylethanol (14 mg/ml); and dextrose (20 50 mg/ml). The pH of the final suspension can be adjusted to range from about pH 5 to pH 7, with a pH of about pH 5.5 being typical.

Another specific example of an aqueous suspension suitable for administration of the compounds via inhalation contains 20 mg/mL Compound or prodrug, 1% (v/v) Polysorbate 80 (TWEEN^{®} 80), 50 mM citrate and/or 0.9% sodium chloride.

For ocular administration, the active compound(s) or prodrug(s) may be formulated as a solution, emulsion, suspension, etc. suitable for administration to the eye. A variety of vehicles suitable for administering compounds to the eye are known in the art. Specific non-limiting examples are described in U.S. Pat. Nos. 6,261,547; 6,197,934; 6,056,950; 5,800,807; 5,776,445; 5,698,219; 5,521,222; 5,403,841; 5,077,033; 4,882,150; and 4,738,851.

For prolonged delivery, the disclosed compound(s) can be formulated as a depot preparation for administration by implantation or intramuscular injection. The active ingredient maybe formulated with suitable polymeric or hydrophobic materials (e.g., as an emulsion in an acceptable oil) or ion exchange resins, or as sparingly soluble derivatives, e.g., as a sparingly soluble salt. Alternatively, transdermal delivery systems manufactured as an adhesive disc or patch which slowly releases the disclosed compound(s) for percutaneous absorption may be used. To this end, permeation enhancers may be used to facilitate transdermal penetration of the active compound(s). Suitable transdermal patches are described in for example, U.S. Pat. Nos. 5,407,713; 5,352,456; 5,332,213; 5,336,168; 5,290,561; 5,254,346; 5,164,189; 5,163,899; 5,088,977; 5,087,240; 5,008,110; and 4,921,475.

Alternatively, other pharmaceutical delivery systems may be employed. Liposomes and emulsions are well-known examples of delivery vehicles that may be used to deliver active compound(s) or prodrug(s). Certain organic solvents, such as dimethylsulfoxide (DMSO), may also be employed, although usually at the cost of greater toxicity. In some embodiments, the disclosed compound(s) as an active ingredient or solvates, N-oxides, pharmaceutically acceptable salts or prodrug(s) thereof, is administered orally in the form of a tablet, e.g., a form of fostamatinib may be administered as TAVALISSE^{®}.

The pharmaceutical compositions may, if desired, be presented in a pack or dispenser device which may contain one or more unit dosage forms containing the active compound(s). The pack may, for example, comprise metal or plastic foil, such as a blister pack. The pack or dispenser device may be accompanied by instructions for administration.

### VII. Dosages

The disclosed compound(s) or a composition thereof, will generally be used in an amount effective to achieve a desired result, for example, in an amount effective to treat or prevent CRS. The compound(s), or compositions thereof, can be administered therapeutically to achieve a therapeutic benefit and/or prophylactically to achieve a prophylactic benefit. Therapeutic benefit means eradication or amelioration of the underlying CRS and/or eradication or amelioration of one or more of the symptoms associated with CRS, such that the patient reports an improvement in feeling or condition, notwithstanding that the patient may still be afflicted with CRS. In some embodiments, indicators of therapeutic improvement and/or successful treatment may include preventing the subject from exhibiting one or more symptoms at a relevant score on the CRS grading scale, such as preventing a subject from exhibiting grade 2 or higher CRS. Additionally, or alternatively, an indicator of therapeutic improvement and/or successful treatment may be a change in grading or severity on the grading scale as discussed herein, such as a change from a score of 4 to a score of 3 or lower, or a change from a score of 3 to a score of 2 or 1. A prophylactic benefit may be achieved by substantially preventing CRS from developing, such as preventing the onset of any symptoms, or preventing one or more symptoms from progressing above grade 1. In some embodiments, prophylactic benefit may mean preventing the subject from exhibiting one or more symptoms at a level of grade 2 or higher.

As known by those of ordinary skill in the art, the preferred dosage of the compound(s) also will depend on various factors, including the age, weight, general health, and severity of the condition of the patient or subject being treated. Dosage also may need to be tailored to the sex of the individual and/or the lung capacity of the individual, when administered by inhalation. Dosage also may be tailored to individuals suffering from more than one condition or those individuals who have additional conditions that affect lung capacity and the ability to breathe normally, for example, emphysema, bronchitis, pneumonia, and respiratory infections. Dosage, and frequency of administration of the disclosed compound(s) or compositions thereof, will also depend on whether the compound(s) are formulated for treatment of acute episodes of CRS or for the prophylactic treatment of CRS. A person or ordinary skill in the art will be able to determine the optimal dose for a particular individual.

The disclosed compound(s), or compositions thereof, can be administered before, during, and/or after therapy that can induce CRS. In one embodiment, the disclosed compound(s), or compositions thereof, is administered within 48 hours before therapy that can induce CRS is to begin, such as within 24, 12, 6, 4, or 2 hours of the therapy. In another embodiment, the disclosed compound(s), or compositions thereof, can be administered during the course of the therapy. In another embodiment the disclosed compound(s), or compositions thereof, can be administered following completion of the therapy, either immediately or shortly following completion of the therapy (e.g., within 24, 48, 72 or 96 hours or 1 week of the completion of therapy). In another embodiment, the disclosed compound(s), or compositions thereof, can be administered during two or more of the time periods consisting of before, during, or after the therapy.

For prophylactic administration, the disclosed compound(s), or compositions thereof, can be administered to a patient or subject at risk of developing CRS. For example, a compound(s), or composition thereof, can be administered to a subject prior to the start of a treating likely to cause CRS, substantially simultaneously with the onset of such a treatment, or subsequent to the treatment being initiated. A compound(s), or compositions thereof, also can be administered prophylactically to individuals who may be repeatedly treated by a treatment that has caused CRS in other individually, even if the subject previously has not developed CRS.

Effective dosages can be estimated initially from *in vitro* assays. For example, an initial dosage for use in subjects can be formulated to achieve a circulating blood or serum concentration of active compound that is at or above an IC₅₀ or EC₅₀ of the particular compound as measured in an *in vitro* assay. Dosages can be calculated to achieve such circulating blood or serum concentrations taking into account the bioavailability of the particular compound. Fingl & Woodbury, "General Principles," In: Goodman and Gilman's The Pharmaceutical Basis of Therapeutics, Chapter 1, pages 1-46, Pergamon Press, and the references cited therein, provide additional guidance concerning effective dosages.

In some embodiments, the disclosed compounds have an EC₅₀ from greater than 0 to 20 µM, such as from greater than 0 to 10 µM, from greater than 0 to 5 µM, from greater than 0 to 1 µM, from greater than 0 to 0.5 µM, or from greater than 0 to 0.1 µM.

Initial dosages can also be estimated from *in vivo* data, such as animal models, including mouse and non-human primate models. CRS animal models are known to persons of ordinary skill in the art, and additional information may be found in Norelli, M., Camisa, B., Barbiera, G. et al. Monocyte-derived IL-1 and IL-6 are differentially required for cytokine-release syndrome and neurotoxicity due to CAR T cells. Nat Med. 2018; 24: 739-748, and Giavridis, T., van der Stegen, S.J.C., Eyquem, J., Hamieh, M., Piersigilli, A., and Sadelain, M. CAR T cell-induced cytokine release syndrome is mediated by macrophages and abated by IL-1 blockade. Nat Med. 2018; 24: 731-738

Dosage amounts of disclosed compounds will typically be in the range of from about greater than 0 mg/kg/day, such as 0.0001 mg/kg/day or 0.001 mg/kg/day or 0.01 mg/kg/day, up to at least about 1000 mg/kg/day, such as up to 100 mg/kg/day, but can be higher or lower, depending upon, among other factors, the activity of the compound, its bioavailability, the mode of administration and various factors discussed herein. More typically, the dosage (or effective amount) may range from about 0.0025 mg/kg to about 1 mg/kg administered at least once per day, such as from 0.01 mg/kg to about 0.5 mg/kg or from about 0.05 mg/kg to about 0.15 mg/kg. The total daily dosage typically ranges from about 0.1 mg/kg to about 5 mg/kg or to about 20 mg/kg per day, such as from 0.5 mg/kg to about 10 mg/kg per day or from about 0.7 mg/kg per day to about 2.5 mg/kg/day. Dosage amounts can be higher or lower depending upon, among other factors, the activity of the compound, its bioavailability, the mode of administration, and various factors discussed above.

Dosage amount and dosage interval can be adjusted for individuals to provide plasma levels of the compound(s) that are sufficient to achieve and/or maintain a desired therapeutic or prophylactic effect. For example, the compounds can be administered once per day, multiple times per day, once per week, multiple times per week (e.g., every other day), one per month, multiple times per month, or once per year, depending upon, amongst other things, the mode of administration, the specific indication being treated, and the judgment of the prescribing physician. Persons of ordinary skill in the art will be able to optimize effective local dosages without undue experimentation. In some embodiments, the amount of the disclosed compound in a composition to be administered, or the amount of the compound to be administered in a method disclosed herein, is a suboptimal dose. As used herein, a suboptimal dose is a dose typically used in a single administration to a patient in monotherapy or in standard of care combination therapies.

Compositions comprising one or more of the disclosed compounds typically comprise from greater than 0 up to 99% of the compound, or compounds, and/or other therapeutic agent by total weight percent. More typically, compositions comprising one or more of the disclosed compounds comprise from about 1 to about 20 total weight percent of the compound and other therapeutic agent, and from about 80 to about 99 weight percent of a pharmaceutically acceptable additive.

Typical daily administrations may be in the range of 100 - 300 mg/day, e.g., 100, 150, 200, 250, or 300 mg/day. Administration may be once or twice daily or more, e.g., 100 or 150 mg BID. Accordingly, pharmaceutical dosage forms comprising a compound disclosed herein may contain from 50 - 300 mg of the disclosed compound, e.g., 50, 100, 150, 200, 250, 300 mg of the disclosed compound. In one, non-limiting embodiment, the dosage is 100 mg of a disclosed compound, such as a form of fostamatinib (e.g., TAVALISSE^{®}), in tablet form. In another, non-limiting embodiment, the dosage is 150 mg of a disclosed compound, such as a form of fostamatinib (e.g., TAVALISSE^{®}), in tablet form.

In one embodiment, the method of the invention is performed by initiating administration of a disclosed compound, such as a form of fostamatinib (e.g., TAVALISSE^{®}), at 100 mg orally twice daily with or without food. After 4 weeks, administration is increases to 150 mg twice daily, if needed, to achieve platelet counts of at least 50 x 109/L as necessary to reduce the risk of bleeding.

Preferably, the compound(s), or compositions thereof, will provide therapeutic or prophylactic benefit without causing substantial toxicity. Toxicity of the compound can be determined using standard pharmaceutical procedures. The dose ratio between toxic and therapeutic (or prophylactic) effect is the therapeutic index. Compounds that exhibit high therapeutic indices are preferred.

### VIII. Examples

### Example 1

### N4-(2,2-dimethyl-4-[(di-tert-butyl phosphonoxy)methyl]-3-oxo-5-pyrido[1,4]oxazin-6-yl)-5-fluoro-N2-(3,4,5-trimethoxyphenyl)-2,4-pyrimidinediamine (Compound 3)

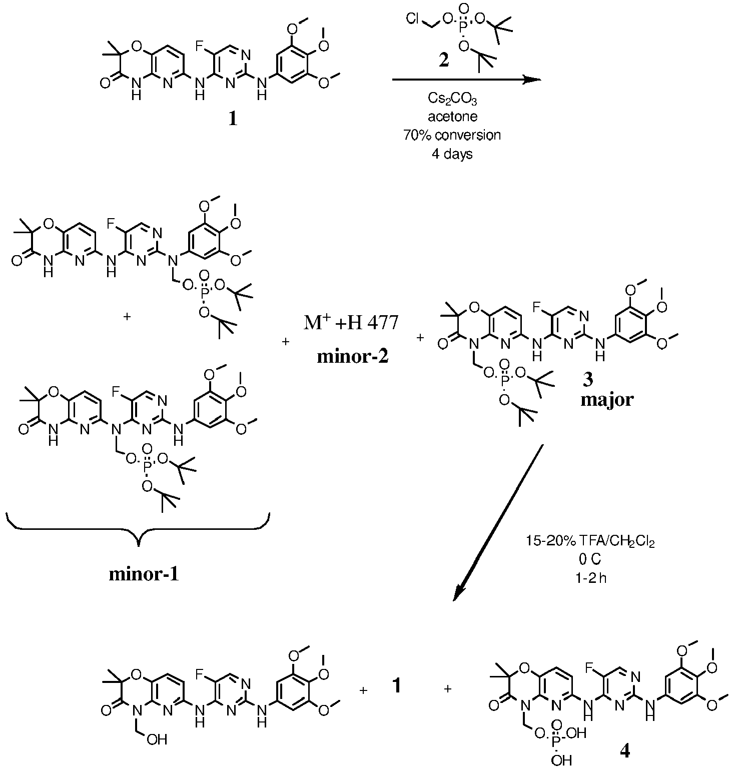

N4-(2,2-dimethyl-3-oxo-4H-5-pyrido[1,4]oxazin-6-yl)-5-fluoro-N2-(3,4,5-trimethoxyphenyl)-2,4-pyrimidinediamine (1, 1.0 g, 2.12 mmol), Cs₂CO₃ (1.0 g, 3.07 mmol) and di-*tert*-butyl chloromethyl phosphate (2, 0.67 g, 2.59 mmol) in acetone (20 mL) was stirred at room temperature under nitrogen atmosphere. Progress of the reaction was monitored by LC/MS. Crude reaction mixture displayed three product peaks with close retention times with M⁺+H 693 (minor-1), 693 (major; 3) and 477 (minor-2) besides starting material (Compound 1). Upon stirring the contents for 4 days (70% consumption), the reaction mixture was concentrated and diluted with water. The resultant pale yellow precipitate formed was collected by filtration and dried. The crude solid was purified by silica gel (pretreated with 10%NEt₃/CH₂Cl₂ followed by eluting with hexanes) column chromatography by gradient elution with 70% EtOAc / hexanes-100% EtOAc). The fractions containing Compound 1 and M⁺+H 693 were collected and concentrated. The resulting crude white solid was subjected to repurification in the similar manner as described previously but by eluting with 30%-50%-75%-100% EtOAc/hexanes. The major product peak with M⁺+H 693 was collected as a white solid (270 mg, 18%) and was characterized as N4-(2,2-dimethyl-4-[(di-tert-butyl phosphonoxy)methyl]-3-oxo-5-pyrido[1,4]oxazin-6-yl)-5-fluoro-N2-(3,4,5-trimethoxyphenyl)-2,4-pyrimidinediamine (Compound 3). ¹H NMR (DMSO-d6): δ 9.21 (s, 1H), 9.17 (s, 1H), 8.16 (d, 1H, J = 2.6 Hz), 7.76 (d, 1H, J = 8.5 Hz), 7.44 (d, 1H, J = 8.5 Hz), 7.02 (s, 2H), 5.78 (d, 1H, J³_{PH} = 6.1 Hz), 3.64 (s, 6H), 3.58 (s, 3H), 1.45 (s, 6H), 1.33 (s, 9H). LCMS: ret. time: 14.70 min.; purity: 95%; MS (*m*/*e*): 693 (MH⁺). ³¹P NMR (DMSO-d6): -11.36.

### N4-(2,2-dimethyl-4-[(dihydrogen phosphonoxy)methyl]-3-oxo-5-pyrido[1,4]oxazin-6-yl)-5-fluoro-N2-(3,4,5-trimethoxyphenyl)-2,4-pyrimidinediamine (Compound 4)

Trifluoroacetic acid (1.5 mL) was added dropwise as a neat for 5 min to N4-(2,2-dimethyl-4-[(di-tert-butyl phosphonoxy)methyl]-3-oxo-5-pyrido[1,4]oxazin-6-yl)-5-fluoro-N2-(3,4,5-trimethoxyphenyl)-2,4-pyrimidinediamine (Compound 3, 120 mg, 0.173 mmol ) dissolved in CH₂Cl₂ (10 mL) at 0°C under nitrogen atmosphere. The contents were allowed to stir for 1.5 h. Progress of the reaction mixture was monitored by LC/MS. After complete consumption of the starting material, reaction mixture was concentrated, dried and triturated with ether. The ethereal layer was decanted and dried to provide the crude solid. LC/MS analysis of the crude displayed three peaks with M⁺+H 581, 471 and 501. The peak corresponding to M⁺+H 581 was collected by preparative HPLC chromatographic purification. The fractions were lyophilised and dried to provide 53 mg (52%) of off white fluffy solid and characterized as N4-(2,2-dimethyl-4-[(dihydrogen phosphonoxy)methyl]-3-oxo-5-pyrido[1,4]oxazin-6-yl)-5-fluoro-N2-(3,4,5-trimethoxyphenyl)-2,4-pyrimidinediamine (Compound 4). ¹H NMR (DMSO-d6): δ 9.21 (br s, 2H), 8.16 (d, 1H, J = 2.6 Hz), 7.93 (d, 1H, J = 8.5 Hz), 7.39 (d, 1H, J = 8.5 Hz), 7.05 (s, 2H), 5.79 (d, 1H, J³_{PH} = 6.6 Hz), 3.67 (s, 6H), 3.59 (s, 3H), 1.44 (s, 6H). LCMS: ret. time: 8.52 min.; purity: 95%; MS (*m*/*e*): 581 (MH⁺). ³¹P NMR (DMSO-d6): -2.17.

### Alternative Synthesis of Prodrug Compound 4

An alternative method of synthesizing prodrug Compound 4 which alleviates the need for column chromatography and HPLC purification is provided below.

### Synthesis of N4-(2,2-dimethyl-4-[(di-tert-butyl phosphonoxy)methyl]-3-oxo-5-pyrido[1,4]oxazin-6-yl)-5-fluoro-N2-(3,4,5-trimethoxyphenyl)-2,4-pyrimidinediamine

### (Compound 3)

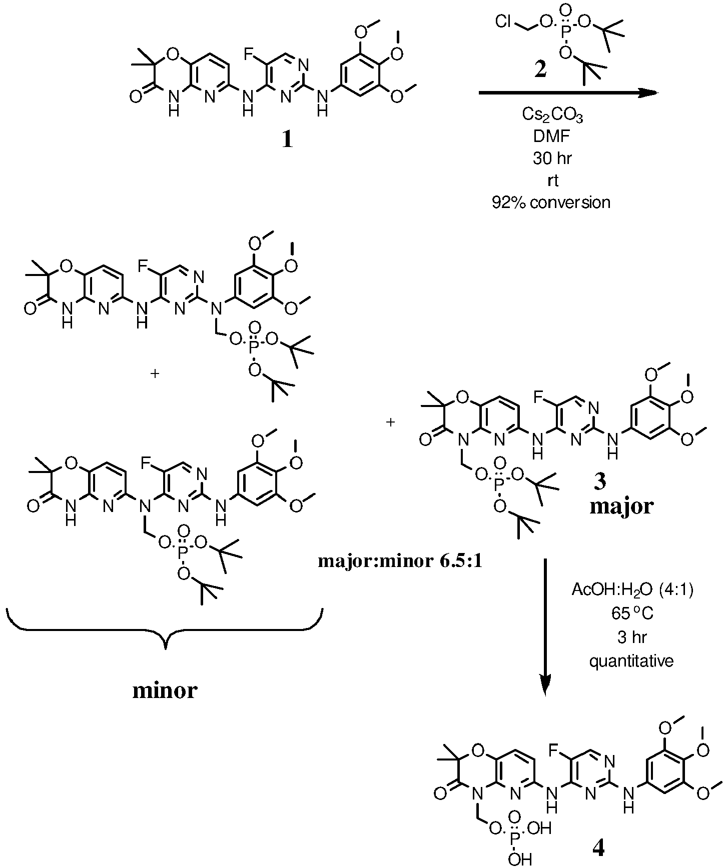

N4-(2,2-dimethyl-3-oxo-4H-5-pyrido[1,4]oxazin-6-yl)-5-fluoro-N2-(3,4,5-trimethoxyphenyl)-2,4-pyrimidinediamine (Compound 1, 19.73 g, 41.97 mmol), Cs₂CO₃ (15.04 g, 46.16 mmol) and di-*tert*-butyl chloromethyl phosphate (13.0 g, 50.38 mmol) in DMF (100 mL) was stirred at room temperature under nitrogen atmosphere. Progress of the reaction was monitored by in process LC/MS. Crude reaction mixture displayed two product peaks (ratio 1:6.5) with close retention times displaying M⁺+H 693 (minor) and 693 (major) besides starting material (Compound 1). Initial yellow reaction mixture turned to olive green as the reaction progressed. Workup is carried out as follows
1). Upon stirring the contents for 30 h (92% consumption), reaction mixture was poured onto ice-water (400 mL) and stirred the contents by adding brine solution (200 mL). Fine yellow tan solid formed was filtered, washed with water and dried overnight.
2). The solid (35 g) was dissolved in MTBE (500 mL) and washed with water (400mL). Aqueous layer was extracted with MTBE (2 X 350 mL) till the absence of UV on TLC. Combined organic layers were dried over anhydrous Na₂SO₄ and decanted.
   Note: step 2 can be done directly, however, DMF extraction back into solution leads to difficulty in the crystallization step.
3). The dark red clear solution was subjected to 10 g of activated charcoal treatment, heated to boil and filtered.
4). The dark red clear solution was concentrated by normal heating to 400 mL of its volume and left for crystallization. The solid crystallized as granules was filtered, crushed the granules to powder, washed with MTBE (400 mL) and dried under high vacuum. See step 7 for the workup of mother liquor. Weight of the solid: 17 g; purity: 90% (Compound 3), 6.26% (Compound 1), 1.8% (minor M+ 693).
5). At this stage solid was taken in 500 ml of ethylether and heated to boil. Cooled and filtered to remove undissolved material. Filtrate was concentrated.
6). Above concentrate was subjected to crystallization in MTBE (300 mL). The white solid formed was filtered, washed with MTBE (100 mL) and dried under high vacuum to provide the desired N4-(2,2-dimethyl-4-[(di-tert-butyl phosphonoxy)methyl]-3-oxo-5-pyrido[1,4]oxazin-6-yl)-5-fluoro-N2-(3,4,5-trimethoxyphenyl)-2,4-pyrimidinediamine (Compound 3) in 97% purity. ¹H NMR (DMSO-d6): δ 9.21 (s, 1H), 9.17 (s, 1H), 8.16 (d, 1H, J = 2.6 Hz), 7.76 (d, 1H, J = 8.5 Hz), 7.44 (d, 1H, J = 8.5 Hz), 7.02 (s, 2H), 5.78 (d, 1H, J³_{PH} = 6.1 Hz), 3.64 (s, 6H), 3.58 (s, 3H), 1.45 (s, 6H), 1.33 (s, 9H). LCMS: ret. time: 14.70 min.; purity: 95%; MS (*m*/*e*): 693 (MH⁺). ³¹P NMR (DMSO-d6): -11.36. Weight of the solid: 15.64 g (yield: 55%); purity: 97% (R935787), 3% (Compound 1).
7). Mother liquor was concentrated and steps 5 and 6 were repeated to provide Compound 3.

### Synthesis of N4-(2,2-dimethyl-4-[(dihydrogen phosphonoxy)methyl]-3-oxo-5-pyrido[1,4]oxazin-6-yl)-5-fluoro-N2-(3,4,5-trimethoxyphenyl)-2,4-pyrimidinediamine

### (Compound 4)

N4-(2,2-dimethyl-4-[(di-tert-butyl phosphonoxy)methyl]-3-oxo-5-pyrido[1,4]oxazin-6-yl)-5-fluoro-N2-(3,4,5-trimethoxyphenyl)-2,4-pyrimidinediamine (Compound 3); (15.0 g, 21.67 mmol) dissolved in AcOH:H₂0 (225 mL, 4:1) was heated at 65 ⁰C (oil bath temp). The progress of the reaction was monitored by in process LC/MS. The reaction mixture transformed to faint tan white solid after 1h of heating. At this point most of Compound 3 converted to mono des t-butyl product. After 3h of heating, consumption of SM and complete conversion of intermediate (mono des t-butylated) to product was observed.

Reaction mixture was cooled, poured onto ice-water (200 mL), stirred for 20 min and filtered. The clear white filter cake was washed with water (600 ml) and acetone (200 mL) successively, dried for 2h followed by drying under high vacuum over P₂O₅ in a desiccator. Weight of the solid: 12.70 g; purity: 97% (Compound 3) and 3% (Compound 1) ¹H NMR indicated acetic acid presence (1:1)

To remove acetic acid, the solid was taken in acetonitrile (300 mL) and concentrated by rotovap vacuum. This process was repeated 2 times with acetonitrile and toluene (3 X 300 mL). The solid obtained was dried under high vacuum at 50 0C.

Finally, the solid was taken in acetone (400 mL), filtered and dried to provide N4-(2,2-dimethyl-4-[(dihydrogen phosphonoxy)methyl]-3-oxo-5-pyrido[1,4]oxazin-6-yl)-5-fluoro-N2-(3,4,5-trimethoxyphenyl)-2,4-pyrimidinediamine (Compound 4). ¹H NMR (DMSO-d6): δ 9.21 (br s, 2H), 8.16 (d, 1H, J = 2.6 Hz), 7.93 (d, 1H, J = 8.5 Hz), 7.39 (d, 1H, J = 8.5 Hz), 7.05 (s, 2H), 5.79 (d, 1H, J³_{PH} = 6.6 Hz), 3.67 (s, 6H), 3.59 (s, 3H), 1.44 (s, 6H). LCMS: ret. time: 8.52 min.; purity: 95%; MS (*m*/*e*): 581 (MH⁺). ³¹P NMR (DMSO-d6):
-2.17.

### Synthesis of N4-(2,2-dimethyl-4-[(dihydrogen phosphonoxy)methyl]-3-oxo-5-pyrido[1,4]oxazin-6-yl)-5-fluoro-N2-(3,4,5-trimethoxyphenyl)-2,4-pyrimidinediamine mono calcium salt (Compound 6)

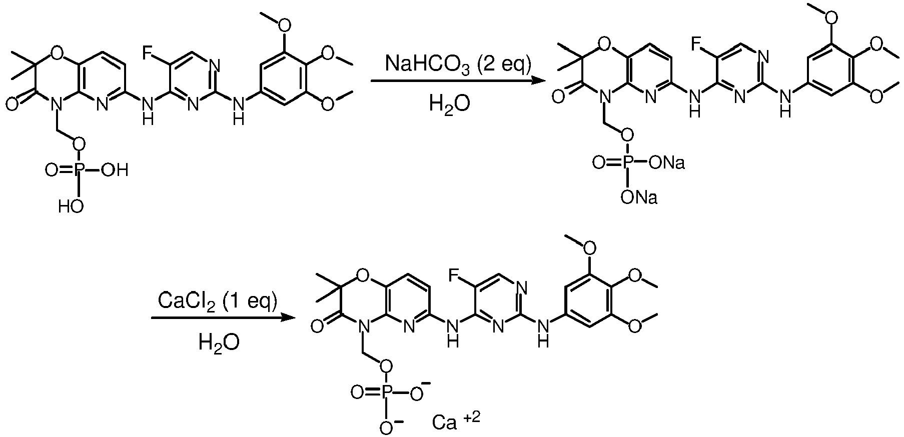

Aqueous (10 mL) NaHCO₃ (0.17 g, 2.02 mmol) solution was added dropwise to a suspension of N4-(2,2-dimethyl-4-[(dihydrogen phosphonoxy)methyl]-3-oxo-5-pyrido[1,4]oxazin-6-yl)-5-fluoro-N2-(3,4,5-trimethoxyphenyl)-2,4-pyrimidinediamine (0.5 g, 0.86 mmol) in water (5 mL) at room temperature while stirring the contents. The clear solution formed was treated with aqueous (10 mL) CaCl₂ (0.11 g in 10 mL water, 0.99 mmol) in a dropwise manner at room temperature. The addition resulted in the precipitation of a white solid from reaction mixture. Upon completion of addition, the contents were stirred for a period of 30 min, filtered, washed with water (40 mL) and dried. The clear white solid was taken in water (30 mL) and heated on a stir plate to boil. The solution was cooled, filtered and dried. The white solid collected and further dried under high vacuo at 80 °C for 32 h to provide 0.41 g (83%) of N4-(2,2-dimethyl-4-[(dihydrogen phosphonoxy)methyl]-3-oxo-5-pyrido[1,4]oxazin-6-yl)-5-fluoro-N2-(3,4,5-trimethoxyphenyl)-2,4-pyrimidinediamine mono calcium salt (Compound 6).

### Synthesis of Prodrug Compound 8

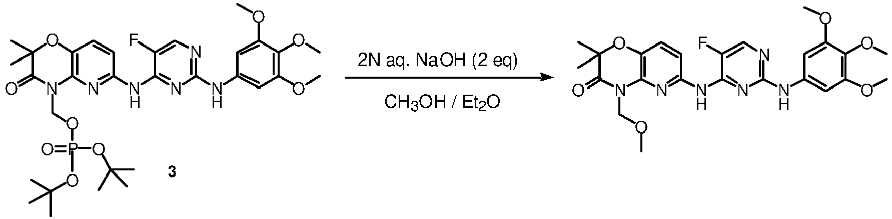

N4-(2,2-dimethyl-4-[(di-tert-butyl phosphonoxy)methyl]-3-oxo-5-pyrido[1,4]oxazin-6-yl)-5-fluoro-N2-(3,4,5-trimethoxyphenyl)-2,4-pyrimidinediamine (prepared as described above) (0.2 g, 0.29 mmol) was added to a mixture of MeOH(5 mL) and Et₂O (5 mL). 2N aq. NaOH (0.023 g, 0.58 mmol) was added at once while stirring the contents at room temperature. Progress of the reaction was monitored by LC/MS. After 8h of stirring, the solid precipitated was filtered and dried to provide N4-(2,2-dimethyl-4-methoxymethyl-3-oxo-5-pyrido[1,4]oxazin-6-yl)-5-fluoro-N2-(3,4,5-trimethoxyphenyl)-2,4-pyrimidinediamine (Compound 8) as a white solid (0.11 g, 74%). ¹H NMR (DMSO-d6): δ 9.47 (s, 1H), 9.15 (s, 1H), 8.16 (d, 1H, J = 3.8 Hz), 7.87 (d, 1H, J = 8.5 Hz), 7.37 (d, 1H, J = 8.5 Hz), 7.03 (s, 2H), 5.40 (s, 2H), 3.66 (s, 6H), 3.59 (s, 3H), 3.27 (s, 3H), 1.44 (s, 6H). LCMS: ret. time: 12.88 min.; purity: 92%; MS (*m*/*e*): 515 (MH⁺).

### Example 5

### In vitro testing for compound effectiveness in the treatment of CRS

### Materials

1. Human PBMC cells (PPA Research Group, Cat No. 15-00021)
2. RPMI media 10% FBS
3. GMCSF (Peprotech, Cat No. 300-03) and IL4 (Peprotech Cat No. 200-04)
4. White clear bottom 96 well plates (Fisher, Cat No. 07-200-587, Corning #3903)
5. Human IL-2 DuoSet ELISA (R&D Systems, Cat No. DY202)
6. Human IL-6 DuoSet ELISA (R&D Systems, Cat No. DY206)
7. Cell Titer Glo reagent (Promega, Cat No. G7573)
8. Dynabeads Human T-Activator CD3/CD28 (Fisher, Cat No. 111.61D)
9. Anti-human CD3 (BD Biosciences, Cat No. 555336)
10. CD28, Clone CD28.2 (Beckman Coulter Inc. Cat No. IM1376)
11. Recombinant Human IL-2 Protein (R&D Systems, Cat No. 202-IL-500)

### Differentiation of DC:

Transfer human PBMC cells (400 million) obtained from the vendor into three T-175 flasks containing 16 ml RPMI media (10% FBS) and incubate for 2 h at 37° C.

After 2 h, remove floating PBL and rinse cell twice with 10 ml of media. Save PBL and media for T cell expansion. Add 16 ml of fresh RPMI media (10% FBS) containing GMCSF (100 ng/ml) and IL4 (20 ng/ml) and keep the flask in 37°C incubator.

After 3 days, add fresh GMCSF (100 ng/ml) and IL4 (20 ng/ml) to the flask and continue the incubation.

### Expansion of T cells:

1. Coat T-175 flask with 16 mls of PBS with 1ug/ml anti-CD3 (16ul of 1mg/ml Stock) and 5ug/ml anti-cd28 (400ul of 200ug/ml stock) for about 2 hours.
2. Spin down and re-suspend 2×E⁸ PBL into 60mls of RPMI media (10% FBS) with 60 µl IL2.
3. Aspirate off coating solution from flask and add cells to stimulation flask.
4. After 3 days knock the stimulation flask to dislodge any cells stuck on the bottom of the flask. Reseed in new T-175 in 60 mls media with 60 µl IL2 at 1xE6 cells/ml.

### CRS Assay:

1. After 4 days, harvest the dendritic cells by spinning down (1000 rpm / 10 min) and aspirate the media. Re-suspend the cells in fresh RPMI media (10% FBS) and plate the cells (25K/well in 50 µl) onto a white clear bottom 96 well plate.
2. Add 100 µl of RPMI media containing 2X concentrated test compound per well to the above cell-culture media (final concentration becomes 1X) and pre-incubate the plates for 1 h at 37° C.
3. After 1 h compound pre-incubation, add 50 µl per well of T cells (1.7k/well) with CD3/CD28 beads (1.7k/well). Incubate the plates at 37° C overnight.
4. Harvest 80 µl of the supernatant from each well for IL6 ELISA and 80 µl of the supernatant for IL2 ELISA. ELISAs are carried out per instructions from R&D Systems. To the remaining 40 µl / well of the cell culture plate add 25 µl of Cell Titer Glo reagent, incubate for 1-2 minutes on a shaker and read the plate for luminescence intensity to determine the compound cytotoxicity.

| **Compound** | **Target** | **Dendritic cells + T cells + CD3/CD28 beads IL6 ELISA (IC₅₀ µM)** | **Dendritic cells + T cells** + **CD3/CD28 beads IL2 ELISA (IC₅₀ µM)** | **Cell Titer-Glo 2** |
|---|---|---|---|---|
| R406 | Syk | 0.119 | 3.14 | 2.53 |
| ACP-196 Acalabrutinib-1 | Btk | 9999 | 9999 | 9999 |
| Cyclosporine A | | 0.023 | 0.009 | 8.49 |

| | | | | |
|---|---|---|---|---|
| NB: IL-6 is primarily produced by the dendritic cells activated by the T cells, and IL-2 is only produced by the activated T cells. | | | | |

## Claims

1. A compound for use in a method for treating and/or preventing cytokine release syndrome (CRS) in a subject experiencing, or at risk of developing, CRS, wherein the compound has a structure according to Formula I 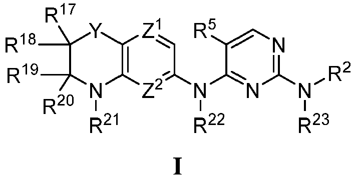 or a salt, solvate, N-oxide or prodrug thereof, wherein:
Y is selected from CH₂, NR²⁴, O, S, S(O) and S(O)₂;
Z¹ and Z² are each, independently of one another, selected from CH and N;
R² is selected from (C₁-C₈) alkyl optionally substituted with one or more of the same or different R⁸ groups, (C₃-C₈) cycloalkyl optionally substituted with one or more of the same or different R⁸ groups, cyclohexyl optionally substituted with one or more of the same or different R⁸ groups, 3-8 membered heterocycloalkyl optionally substituted with one or more of the same or different R⁸ groups, (C₆-C₁₄) aryl optionally substituted with one or more of the same or different R⁸ groups, phenyl optionally substituted with one or more of the same or different R⁸ groups and 5-15 membered heteroaryl optionally substituted with one or more of the same or different R⁸ groups;
R⁵ is selected from halo, cyano, nitro, or trihalomethyl;
each R⁸ independently is selected from R^{a}, R^{b}, R^{a} substituted with one or more of the same or different R^{a} or R^{b}, -OR^{a} substituted with one or more of the same or different R^{a} or R^{b}, -B(OR^{a})₂, -B(NR^{c}R^{c})₂, -(CH₂)*ₘ*-R^{b}, -(CHR^{a})*ₘ*-R^{b}, -O-(CH₂)*ₘ*-R^{b}, -S-(CH₂)*ₘ*-R^{b}, -O-CHR^{a}R^{b}, -O-CR^{a}(R^{b})₂, -O-(CHR^{a})*ₘ*-R^{b}, -O- (CH₂)*ₘ*-CH[(CH₂)*ₘ*R^{b}]R^{b}, -S-(CHR^{a})*ₘ*-R^{b}, -C(O)NH-(CH₂)*ₘ-*R^{b}, -C(O)NH-(CHR^{a})*ₘ*-R^{b}, -O-(CH₂)*ₘ*-C(O)NH-(CH₂)*ₘ*-R^{b}, -S-(CH₂)*ₘ*-C(O)NH-(CH₂)*ₘ*-R^{b}, -O-( CHR^{a})*ₘ*-C(O)NH-(CHR^{a})*ₘ*-R^{b}, -S-(CHR^{a})*ₘ*-C(O)NH-(CHR^{a})*ₘ*-R^{b}, -NH-(CH₂)*ₘ*-R^{b}, -NH-(CHR^{a}) *ₘ*-R^{b}, -NH[(CH₂)*ₘ*R^{b}], -N[(CH₂)*ₘ*R^{b}]₂, -NH-C(O)-NH-(CH₂)*ₘ*-R^{b}, -NH-C(O)-(CH₂)*ₘ*-CHR^{b}R^{b} and -NH-(CH₂)*ₘ*-C(O)-NH-(CH₂)*ₘ*-R^{b};
R¹⁷ is selected from hydrogen, halogen, or (C₁-C₈) alkyl;
R¹⁸ is selected from hydrogen, halogen, (C₁-C₈) alkyl;
or, alternatively, R¹⁸ may be taken together with R¹⁷ to form an oxo (=O) group or, together with the carbon atom to which they are attached, a spirocycle containing from 3 to 7 carbon atoms;
R¹⁹ is selected from hydrogen, or (C₁-C₈) alkyl;
R²⁰ is selected from hydrogen, or (C₁-C₈) alkyl;
or, alternatively, R²⁰ may be taken together with R¹⁹ to form an oxo (=O) group or, together with the carbon atom to which they are attached, a spirocycle containing from 3 to 7 carbon atoms;
each R^{a} is, independently of the others, selected from hydrogen, (C₁-C₈) alkyl, (C₃-C₈) cycloalkyl, cyclohexyl, (C₄-C₁₁) cycloalkylalkyl, (C₆-C₁₀) aryl, phenyl, (C₇-C₁₆) arylalkyl, benzyl, 2-6 membered heteroalkyl, 3-8 membered heterocycloalkyl, 4-11 membered heterocycloalkylalkyl, 5-10 membered heteroaryl and 6-16 membered heteroarylalkyl;
each R^{b} is independently selected from =O, -OR^{a}, (C₁-C₃) haloalkyloxy, =S, -SR^{a}, =NR^{a}, =NOR^{a}, -NR^{c}R^{c}, halogen, -CF₃, -CN, -NC, -OCN, -SCN, -NO, -NO₂, =N₂, -N₃, -S(O)R^{a}, -S(O)₂R^{a}, -S(O)₂OR^{a}, -S(O)NR^{c}R^{c}, -S(O)₂NR^{c}R^{c}, -OS(O)R^{a}, -OS(O)₂R^{a}, -OS(O)₂OR^{a}, -OS(O)₂NR^{c}R^{c}, -C(O)R^{a}, -C(O)OR^{a}, -C(O)NR^{c}R^{c}, -C(NH)NR^{c}R^{c}, -C(NR^{a})NR^{c}R^{c}, -C(NOH)R^{a}, -C(NOH)NR^{c}R^{c}, -OC(O)R^{a}, -OC(O)OR^{a}, -OC(O)NR^{c}R^{c}, -OC(NH)NR^{c}R^{c}, -OC(NR^{a})NR^{c}R^{c}, -[N HC(O)]*ₙ*R^{a}, -[NR^{a}C(O)]*ₙ*R^{a}, -[NHC(O)]*ₙ*OR^{a}, -[NR^{a}C(O)]*ₙ*OR^{a}, -[NHC(O)]*ₙ*R^{c}R^{c}, -[NR^{a}C(O)]*ₙ* NR^{c}R^{c}, -[NHC(NH)]*ₙ*NR^{c}R^{c} or -[NR^{a}C(NR^{a})]*_{b}*NR^{c}R^{c};
each R^{c} is, independently of the others, selected from R^{a} or an amino-protecting group selected from formyl, acetyl, trifluoroacetyl, benzyl, benzyloxycarbonyl, tert-butoxycarbonyl, trimethylsilyl, 2-trimethylsilyl-ethanesulfonyl, trityl and substituted trityl groups, allyloxycarbonyl, 9-fluorenylmethyloxycarbonyl, or nitro-veratryloxycarbonyl;
or, alternatively, the two R^{c} bonded to the same nitrogen atom are taken together with that nitrogen atom to form a 5 to 8-membered heterocycloalkyl or heteroaryl which may optionally include one or more of the same or different additional heteroatoms and which may optionally be substituted with one or more of the same or different R^{a} groups;
R²¹, R²² and R²³ are each, independently of one another, selected from hydrogen or phosphonooxyalkyl;
R²⁴ is selected from hydrogen, (C₁-C₈) alkyl, or phosphonooxyalkyl;
each *m* is, independently of the others, an integer from 1 to 3; and
each *n* is, independently of the others, an integer from 0 to 3.

2. The compound for use according to claim 1, wherein at least one of R²¹, R²², R²³ and R²⁴ is phosphonooxyalkyl.

3. The compound for use according to claim 1 or claim 2, wherein at least one of R²¹, R²², R²³ and R²⁴ is hydrogen.

4. The compound for use according to any one of claims 1-3, wherein R²¹ is phosphonooxyalkyl, and R²², R²³ and R²⁴ are hydrogen.

5. The compound for use according to any one of claims 1-4, wherein the compound has a formula selected from 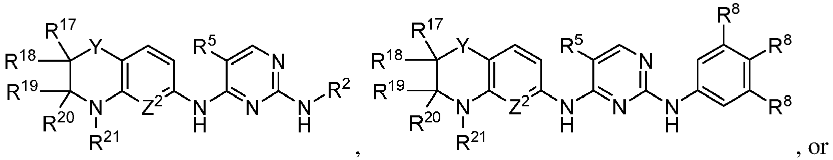 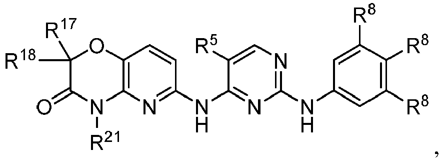 or a salt, solvate, N-oxide or prodrug thereof.

6. The compound for use according to any one of claims 1-5, wherein the compound has a formula 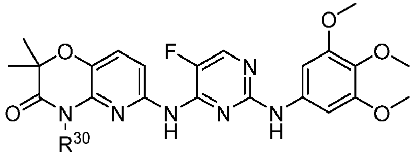 or a salt, solvate, N-oxide or prodrug thereof, where R³⁰ is H or phosphonooxyalkyl.

7. The compound for use according to any one of claims 1-6, wherein the compound is 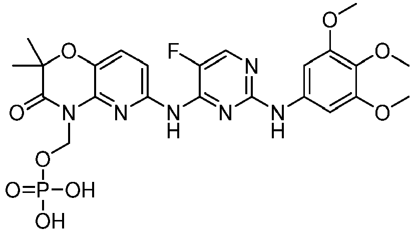 or a salt and/or solvate thereof.

8. The compound for use according to any one of claims 1-7, wherein the compound is

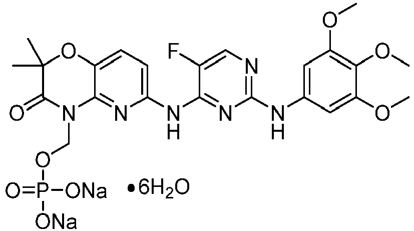

9. The compound for use according to any one of claims 1-6, wherein the compound is

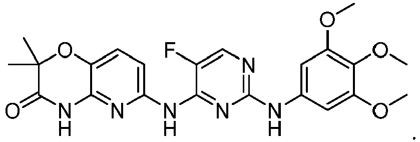

10. The compound for use according to any one of claims 1-9, wherein administering the compound ameliorates a sign or symptom of CRS, compared to the severity of the sign or symptom prior to administration of the compound;
preferably wherein the sign or symptom is a fever.

11. The compound for use according to any one of claims 1-10, wherein administering comprises:
administering to a subject that has previously be administered a first therapy for which CRS is a known, suspected, or potential side effect; or
administering to a subject who will be, or is concurrently being, administered a first therapy for which CRS is a known, suspected, or potential side effect.

12. The compound for use according to claim 11, wherein the first therapy comprises a cell therapy;
preferably wherein the cell therapy comprises chimeric antigen receptor (CAR)-expressing therapy, a transgenic receptor therapy, or a combination thereof.

13. The compound for use according to any one of claims 1-12, wherein administering the compound further comprises administering a second therapeutic agent;
preferably wherein the second therapeutic agent is a steroid, an anti-inflammatory agent, an immunosuppressant, or a combination thereof.

14. The compound for use according to claim 13, wherein:
the steroid is alclomethasone, algestone, beclomethasone, betamethasone, budesonide, clobetasol, clobetasone, clocortolone, cloprednol, corticosterone, cortisone, cortivazol, deflazacort, desonide, desoximethasone, dexamethasone, diflorasone, diflucortolone, difluprednate, enoxolone, fluazacort, flucloronide, fludrocortisone, flumethasone, flunisolide, fluocinolone, fluocinonide, fluocortin, fluocortolone, fluorometholone, fluperolone, fluprednidene, fluprednisolone, flurandrenolide, fluticasone, formocortal, halcinonide, halobetasol, halometasone, halopredone, hydrocortamate, hydrocortisone, loteprednol etabonate, mazipredone, medrysone, meprednisone, methylprednisolone, mometasone, paramethasone, prednicarbate, prednisolone, prednisone, prednival, prednylidene, rimexolone, tixocortol, triamcinolone, or any combination thereof;
the anti-inflammatory agent is an amino salicylate, cyclooxygenase inhibitor, diclofenac, etodolac, famotidine, fenoprofen, flurbiprofen, ketoprofen, ketorolac, ibuprofen, indomethacin, meclofenamate, mefenamic acid, meloxicam, nambumetone, naproxen, oxaprozin, piroxicam, salsalate, sulindac, tolmetin, or a combination thereof; or
the immunosuppressant is mercaptopurine, a corticosteroid, an alkylating agent, a calcineurin inhibitor, an inhibitor of inosine monophosphate dehydrogenase (IMPDH), an agents designed to suppress cellular immunity while leaving the recipient's humoral immunologic response intact, or a combination thereof.

15. The compound for use according to claim 13, wherein the second therapeutic is dexamethasone or prednisone, or a combination thereof.

## Patentansprüche

1. Verbindung zur Verwendung in einem Verfahren zur Behandlung und/oder Vorbeugung des Zytokinfreisetzungssyndroms (CRS) bei einem Subjekt, das an CRS leidet oder bei dem die Gefahr besteht, CRS zu entwickeln, wobei die Verbindung eine Struktur gemäß Formel I aufweist 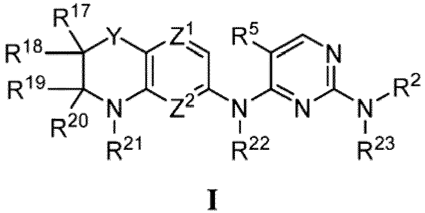 oder ein Salz, Solvat, N-Oxid oder Prodrug davon, wobei:
Y aus CH₂, NR²⁴, O, S, S(O) und S(O)₂ ausgewählt ist;
Z¹ und Z² jeweils unabhängig voneinander aus CH und N ausgewählt sind;
R² ausgewählt ist aus (C₁-C₈)-Alkyl, das optional mit einer oder mehreren der gleichen oder verschiedenen R⁸-Gruppen substituiert ist, (C₃-C₈)-Cycloalkyl, das optional mit einer oder mehreren der gleichen oder verschiedenen R⁸-Gruppen substituiert ist, Cyclohexyl, das optional mit einer oder mehreren der gleichen oder verschiedenen R⁸-Gruppen substituiert ist, 3- bis 8-gliedrigem Heterocycloalkyl, das optional mit einer oder mehreren der gleichen oder verschiedenen R⁸-Gruppen substituiert ist, (C₆-C₁₄)-Aryl, das optional mit einer oder mehreren der gleichen oder verschiedenen R⁸-Gruppen substituiert ist, Phenyl, das optional mit einer oder mehreren der gleichen oder verschiedenen R⁸-Gruppen substituiert ist und 5- bis 15-gliedrigem Heteroaryl, das optional mit einer oder mehreren der gleichen oder verschiedenen R⁸-Gruppen substituiert ist;
R⁵ aus Halogen, Cyano, Nitro oder Trihalogenmethyl ausgewählt ist;
jedes R⁸ unabhängig ausgewählt ist aus R^{a}, R^{b}, R^{a}, das mit einem oder mehreren der gleichen oder verschiedenen R^{a} oder R^{b} substituiert ist, -OR^{a}, das mit einem oder mehreren der gleichen oder verschiedenen R^{a} oder R^{b} substituiert ist, -B(OR^{a})₂, -B(NR^{c}R^{c})₂, -(CH₂)*ₘ*-R^{b}, - (CHR^{a})*ₘ*-R^{b}, -O-(CH₂)*ₘ*-R^{b}, -S-(CH₂)*ₘ*-R^{b}, -O-CHR^{a}R^{b}, -O-CR^{a}(R^{b})₂, -O-(CHR^{a})*ₘ*-R^{b}, -O-(CH₂)*ₘ*-CH[(CH₂)*ₘ*R^{b}]R^{b}, -S-(CHR^{a})*ₘ*-R^{b}, -C(O)NH-(CH₂)*ₘ*-R^{b}, -C(O)NH-(CHR^{a})*ₘ*-R^{b}, -O-(CH₂)*ₘ*-C(O)NH-(CH₂)*ₘ*-R^{b}, -S-(CH₂)*ₘ*-C(O)NH-(CH₂)*ₘ*-R^{b}, -O-(CHR^{a})*ₘ*-C(O)NH-(CHR^{a})*ₙ-*R^{b}, -S-(CHR^{a})*ₘ*-C(O)NH-(CHR^{a})*ₘ*-R^{b}, -NH-(CH₂)*ₘ*-R^{b}, -NH-(CHR^{a})*ₘ*-R^{b}, -NH[(CH₂)*ₘ*R^{b}], - N[(CH₂)*ₘ*R^{b}]₂, -NH-C(O)-NH-(CH₂)*ₘ*-R^{b}, -NH-C(O)-(CH₂)*ₘ*-CHR^{b}R^{b} und -NH-(CH₂)*ₘ*-C(O)-NH-(CH₂)*ₘ*-R^{b} ;
R¹⁷ aus Wasserstoff, Halogen oder (C₁-C₈)-Alkyl ausgewählt ist;
R¹⁸ aus Wasserstoff, Halogen, (C₁-C₈)-Alkyl ausgewählt ist;
oder alternativ R¹⁸ mit R¹⁷ zusammengenommen werden kann, um eine Oxo (=O)-Gruppe zu bilden, oder zusammen mit dem Kohlenstoffatom, an das sie gebunden sind, einen Spirocyclus mit 3 bis 7 Kohlenstoffatomen;
R¹⁹ aus Wasserstoff oder (C₁-C₈)-Alkyl ausgewählt ist;
R²⁰ aus Wasserstoff oder (C₁-C₈)-Alkyl ausgewählt ist;
oder alternativ R²⁰ mit R¹⁹ zusammengenommen werden kann, um eine Oxo (=O)-Gruppe zu bilden, oder zusammen mit dem Kohlenstoffatom, an das sie gebunden sind, einen Spirocyclus mit 3 bis 7 Kohlenstoffatomen;
jedes R^{a} unabhängig von den anderen ausgewählt ist aus Wasserstoff, (C₁-C₈)-Alkyl, (C₃-C₈)-Cycloalkyl, Cyclohexyl, (C₄-C₁₁)-Cycloalkylalkyl, (C₆-C₁₀)-Aryl, Phenyl, (C₇-C₁₆)-Arylalkyl, Benzyl, 2- bis 6-gliedrigem Heteroalkyl, 3- bis 8-gliedrigem Heterocycloalkyl, 4-bis 11-gliedrigem Heterocycloalkylalkyl, 5- bis 10-gliedrigem Heteroaryl und 6- bis 16-gliedrigem Heteroarylalkyl;
jedes R^{b} unabhängig ausgewählt ist aus =O, -OR^{a}, (C₁-C₃)-Haloalkyloxy, =S, -SR^{a}, =NR^{a}, =NOR^{a}, -NR^{c}R^{c}, Halogen, -CF₃, -CN, -NC, -OCN, -SCN, -NO, -NO₂, =N₂, -N₃, -S(O)R^{a}, -S(O)₂R^{a}, -S(O)₂OR^{a}, -S(O)NR^{c}R^{c}, -S(O)₂NR^{c}R^{c}, -OS(O)R^{a}, -OS(O)₂R^{a}, -OS(O)₂OR^{a}, - OS(O)₂NR^{c}R^{c}, -C(O)R^{a}, -C(O)OR^{a}, -C(O)NR^{c}R^{c}, -C(NH)NR^{c}R^{c}, -C(NR^{a})NR^{c}R^{c}, -C(NOH)R^{a}, -C(NOH)NR^{c}R^{c}, -OC(O)R^{a}, -OC(O)OR^{a}, -OC(O)NR^{c}R^{c}, -OC(NH)NR^{c}R^{c}, -OC(NR^{a})NR^{c}R^{c}, - [NHC(O)]*ₙ*R^{a}, -[NR^{a}C(O)]*ₙ*R^{a}, -[NHC(O)]*ₙ*OR^{a}, -[NR^{a}C(O)]*ₙ*OR^{a}, -[NHC(O)]*ₙ*NR^{c}R^{c}, - [NR^{a}C(O)]*ₙ*NR^{c}R^{c}, -[NHC(NH)]*ₙ*NR^{c}R^{c} oder -[NR^{a}C(NR^{a})]*ₙ*NR^{c}R^{c};
jedes R^{c} unabhängig von den anderen ausgewählt ist aus R^{a} oder einer Aminoschutzgruppe, ausgewählt aus Formyl, Acetyl, Trifluoracetyl, Benzyl, Benzyloxycarbonyl, *tert*-Butoxycarbonyl, Trimethylsilyl, 2-Trimethylsilyl-ethansulfonyl, Trityl und substituierten Tritylgruppen, Allyloxycarbonyl, 9-Fluorenylmethyloxycarbonyl oder Nitroveratryloxycarbonyl;
oder alternativ die beiden an dasselbe Stickstoffatom gebundenen R^{c} mit diesem Stickstoffatom zusammengenommen werden, um ein 5- bis 8-gliedriges Heterocycloalkyl oder Heteroaryl zu bilden, das optional ein oder mehrere der gleichen oder verschiedenen zusätzlichen Heteroatome enthalten kann und das optional mit einer oder mehreren der gleichen oder verschiedenen R^{a}-Gruppen substituiert sein kann;
R²¹, R²² und R²³ jeweils unabhängig voneinander aus Wasserstoff oder Phosphonooxyalkyl ausgewählt sind;
R²⁴ aus Wasserstoff, (C₁-C₈)-Alkyl oder Phosphonooxyalkyl ausgewählt ist;
jedes *m* unabhängig von den anderen eine Ganzzahl von 1 bis 3 ist; und
jedes n unabhängig von den anderen eine Ganzzahl von 0 bis 3 ist.

2. Verbindung zur Verwendung nach Anspruch 1, wobei mindestens einer von R²¹, R²², R²³ und R²⁴ Phosphonooxyalkyl ist.

3. Verbindung zur Verwendung nach Anspruch 1 oder Anspruch 2, wobei mindestens einer von R²¹, R²², R²³ und R²⁴ Wasserstoff ist.

4. Verbindung zur Verwendung nach einem der Ansprüche 1 bis 3, wobei R²¹ Phosphonooxyalkyl ist und R²², R²³ und R²⁴ Wasserstoff sind.

5. Verbindung zur Verwendung nach einem der Ansprüche 1 bis 4, wobei die Verbindung eine Formel hat, die ausgewählt ist aus 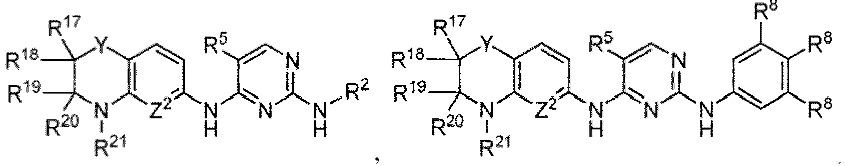 oder 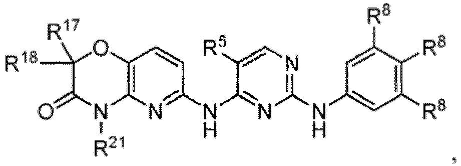 oder ein Salz, Solvat, N-Oxid oder Prodrug davon.

6. Verbindung zur Verwendung nach einem der Ansprüche 1 bis 5, wobei die Verbindung eine Formel 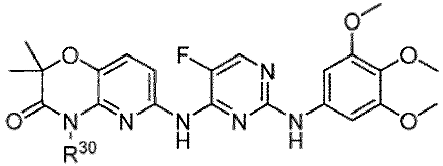 hat, oder ein Salz, Solvat, N-Oxid oder Prodrug davon, wobei R³⁰ H oder Phosphonooxyalkyl ist.

7. Verbindung zur Verwendung nach einem der Ansprüche 1 bis 6, wobei die Verbindung 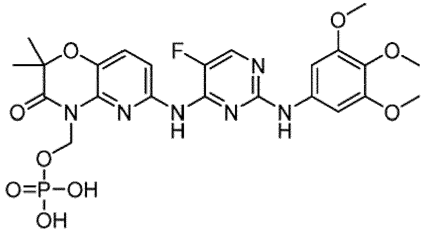 oder ein Salz und/oder Solvat davon ist.

8. Verbindung zur Verwendung nach einem der Ansprüche 1 bis 7, wobei die Verbindung 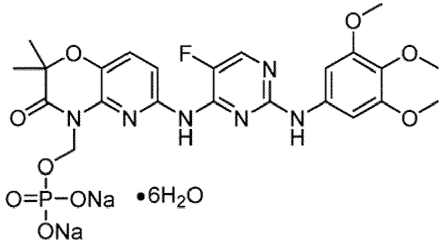 ist.

9. Verbindung zur Verwendung nach einem der Ansprüche 1 bis 6, wobei die Verbindung 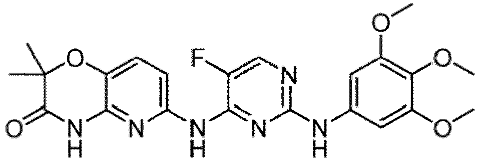 ist.

10. Verbindung zur Verwendung nach einem der Ansprüche 1 bis 9, wobei die Verabreichung der Verbindung ein Anzeichen oder Symptom von CRS im Vergleich zum Schweregrad des Anzeichens oder Symptoms vor der Verabreichung der Verbindung bessert;
wobei das Anzeichen oder Symptom vorzugsweise ein Fieber ist.

11. Verbindung zur Verwendung nach einem der Ansprüche 1 bis 10, wobei die Verabreichung Folgendes umfasst:
Verabreichung an ein Subjekt, dem zuvor eine erste Therapie verabreicht wurde, für die CRS eine bekannte, vermutete oder potenzielle Nebenwirkung ist; oder
Verabreichung an ein Subjekt, dem eine erste Therapie verabreicht werden wird, für die CRS eine bekannte, vermutete oder potenzielle Nebenwirkung ist, oder das gleichzeitig mit dieser Therapie behandelt wird.

12. Verbindung zur Verwendung nach Anspruch 11, wobei die erste Therapie eine Zelltherapie umfasst;
wobei vorzugsweise die Zelltherapie eine chimäre Antigenrezeptor (CAR)-exprimierende Therapie, eine transgene Rezeptortherapie oder eine Kombination davon umfasst.

13. Verbindung zur Verwendung nach einem der Ansprüche 1 bis 12, wobei die Verabreichung der Verbindung ferner die Verabreichung eines zweiten therapeutischen Mittels umfasst;
wobei vorzugsweise das zweite therapeutische Mittel ein Steroid, ein entzündungshemmendes Mittel, ein Immunsuppressivum oder eine Kombination davon ist.

14. Verbindung zur Verwendung nach Anspruch 13, wobei:
das Steroid Alclomethason, Algeston, Beclomethason, Betamethason, Budesonid, Clobetasol, Clobetason, Clocortolon, Cloprednol, Corticosteron, Cortison, Cortivazol, Deflazacort, Desonid, Desoximethason, Dexamethason, Diflorason, Diflucortolon, Difluprednat, Enoxolon, Fluazacort, Flucloronid, Fludrocortison, Flumethason, Flunisolid, Fluocinolon, Fluocinonid, Fluocortin, Fluocortolon, Fluorometholon, Fluperolon, Flupredniden, Fluprednisolon, Flurandrenolid, Fluticason, Formocortal, Halcinonid, Halobetasol, Halometason, Halopredon, Hydrocortamat, Hydrocortison, Loteprednoletabonat, Mazipredon, Medryson, Meprednison, Methylprednisolon, Mometason, Paramethason, Prednicarbat, Prednisolon, Prednisone, Prednival, Prednyliden, Rimexolon, Tixocortol, Triamcinolon oder eine Kombination davon ist;
das entzündungshemmende Mittel ein Aminosalicylat, ein Cyclooxygenasehemmer, Diclofenac, Etodolac, Famotidin, Fenoprofen, Flurbiprofen, Ketoprofen, Ketorolac, Ibuprofen, Indomethacin, Meclofenamat, Mefenaminsäure, Meloxicam, Nambumeton, Naproxen, Oxaprozin, Piroxicam, Salsalat, Sulindac, Tolmetin oder eine Kombination davon ist; oder
das Immunsuppressivum Mercaptopurin, ein Kortikosteroid, ein Alkylierungsmittel, ein Calcineurinhemmer, ein Inosinmonophosphat-Dehydrogenase (EMPDH)-Hemmer, ein Mittel zur Unterdrückung der zellulären Immunität, wobei die humorale Immunantwort des Empfängers intakt bleibt, oder eine Kombination davon ist.

15. Verbindung zur Verwendung nach Anspruch 13, wobei das zweite Therapeutikum Dexamethason oder Prednison oder eine Kombination davon ist.

## Revendications

1. Composé pour l'utilisation dans un procédé pour le traitement et/ou la prévention de syndrome de libération de cytokines (CRS) chez un sujet atteint du CRS, ou à risque de le développer, dans lequel le composé a une structure selon la formule I 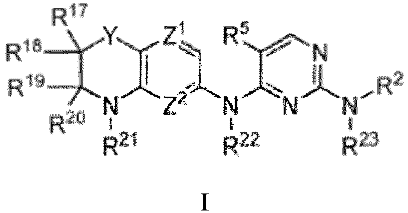 ou un sel, solvate, N-oxyde ou promédicament de celui-ci, dans lequel :
Y est sélectionné parmi CH₂, NR²⁴, O, S, S(O) et S(O)₂ ;
Z¹ et Z² sont chacun, indépendamment l'un de l'autre, sélectionnés parmi CH etN;
R² est sélectionné parmi alkyle (C₁-C₈) optionnellement substitué avec un ou plusieurs des mêmes ou de différents groupes R⁸, cycloalkyle (C₃-C₈) optionnellement substitué avec un ou plusieurs des mêmes ou de différents groupes R⁸, cyclohexyle optionnellement substitué avec un ou plusieurs des mêmes ou de différents groupes R⁸, hétérocycloalkyle, à 3 à 8 membres, optionnellement substitué avec un ou plusieurs des mêmes ou de différents groupes R⁸, aryle (C₆-C₁₄) optionnellement substitué avec un ou plusieurs des mêmes ou de différents groupes R⁸, phényle optionnellement substitué avec un ou plusieurs des mêmes ou de différents groupes R⁸ et hétéroaryle, à 5 à 15 membres, optionnellement substitué avec un ou plusieurs des mêmes ou de différents groupes R⁸ ;
R⁵ est sélectionné parmi halo, cyano, nitro, ou trihalométhyle ;
chaque R⁸ indépendamment est sélectionné parmi R^{a}, R^{b}, R^{a} substitué avec un ou plusieurs des mêmes ou de différents R^{a} ou R^{b}, -OR^{a} substitué avec un ou plusieurs des mêmes ou de différents R^{a} ou R^{b}, -B(OR^{a})₂, -B(NR^{c}R^{c})₂, -(CH₂)*ₘ*-R^{b}, - (CHR^{a})*ₘ*-R^{b}, -O-(CH₂)*ₘ*-R^{b}, -S-(CH₂)*ₘ*-R^{b}, -O-CHR^{a}R^{b}, -O-CR^{a}(R^{b})₂, -O-(CHR^{a})*ₘ-*R^{b}, -O-(CH₂)*ₘ*-CH[(CH₂)*ₘ*R^{b}]R^{b}, -S-(CHR^{a})*ₘ*-R^{b}, -C(O)NH-(CH₂)*ₘ*-R^{b}, -C(O)NH-(CHR^{a})*ₘ*-R^{b}, -O-(CH₂)*ₘ*-C(O)NH-(CH₂)*ₘ*-R^{b}, -S-(CH₂)*ₘ*-C(O)NH-(CH₂)*ₘ*-R^{b}, -O-(CHR^{a})*ₘ*-C(O)NH-(CHR^{a})*ₘ*-R^{b}, -S-(CHR^{a})*ₘ*-C(O)NH-(CHR^{a})*ₘ*-R^{b}, -NH-(CH₂)*ₘ*-R^{b}, -NH-(CHR^{a})*ₘ*-R^{b}, -NH[(CH₂)*ₘ*R^{b}], -N[(CH₂)*ₘ*R^{b}]₂, -NH-C(O)-NH-(CH₂)*ₘ*-R^{b}, -NH-C(O)-(CH₂)*ₘ*-CHR^{b}R^{b} et -NH-(CH₂)*ₘ*-C(O)-NH-(CH₂)*ₘ*-R^{b} ;
R¹⁷ est sélectionné parmi hydrogène, halogène, ou alkyle (C₁-C₈) ;
R¹⁸ est sélectionné parmi hydrogène, halogène, alkyle (C₁-C₈) ;
ou, en variante, R¹⁸ peut être pris conjointement avec R¹⁷ pour former un groupe oxo (=O) ou, conjointement avec l'atome de carbone auquel ils sont attachés, un spirocycle contenant de 3 à 7 atomes de carbone ;
R¹⁹ est sélectionné parmi hydrogène, ou alkyle (C₁-C₈) ;
R²⁰ est sélectionné parmi hydrogène, ou alkyle (C₁-C₈) ;
ou, en variante, R²⁰ peut être pris conjointement avec R¹⁹ pour former un groupe oxo (=O) ou, conjointement avec l'atome de carbone auquel ils sont attachés, un spirocycle contenant de 3 à 7 atomes de carbone ;
chaque R^{a} est, indépendamment des autres, sélectionné parmi hydrogène, alkyle (C₁-C₈), cycloalkyle (C₃-C₈), cyclohexyle, cycloalkylalkyle (C₄-C₁₁), aryle (C₆-C₁₀), phényle, arylalkyle (C₇-C₁₆), benzyle, hétéroalkyle à 2 à 6 membres, hétérocycloalkyle à 3 à 8 membres, hétérocycloalkylalkyle à 4 à 11 membres, hétéroaryle à 5 à 10 membres et hétéroarylalkyle à 6 à 16 membres ;
chaque R^{b} est indépendamment sélectionné parmi =O, -OR^{a}, haloalkyloxy (C₁-C₃), =S, -SR^{a}, =NR^{a}, =NOR^{a}, -NR^{c}R^{c}, halogène, -CF₃, -CN, -NC, - OCN, -SCN, -NO, -NO₂, =N₂, -N₃, -S(O)R^{a}, -S(O)₂R^{a}, -S(O)₂OR^{a}, -S(O)NR^{c}R^{c}, - S(O)₂NR^{c}R^{c}, -OS(O)R^{a}, -OS(O)₂R^{a}, -OS(O)₂OR^{a}, -OS(O)₂NR^{c}R^{c}, -C(O)R^{a}, - C(O)OR^{a}, -C(O)NR^{c}R^{c}, -C(NH)NR^{c}R^{c}, -C(NR^{a})NR^{c}R^{c}, -C(NOH)R^{a}, - C(NOH)NR^{c}R^{c}, -OC(O)R^{a}, -OC(O)OR^{a}, -OC(O)NR^{c}R^{c}, -OC(NH)NR^{c}R^{c}, - OC(NR^{a})NR^{c}R^{c}, -[NHC(O)]*ₙ*R^{a}, -[NR^{a}C(O)]*ₙ*R^{a}, -NHC(O)]*ₙ*OR^{a}, -[NR^{a}C(O)]*ₙ*OR^{a}, - [NHC(O)]*ₙ*NR^{c}R^{c}, -[NR^{a}C(O)]*ₙ*NR^{c}R^{c}, -[NHC(NH)]*ₙ*NR^{c}R^{c} ou-[NR^{a}C(NR^{a})]*ₙ*NR^{c}R^{c} ;
chaque R^{c} est, indépendamment des autres, sélectionné parmi R^{a} ou un groupe amino-protecteur sélectionné parmi formyle, acétyle, trifluoroacétyle, benzyle, benzyloxycarbonyle, tert-butoxycarbonyle, triméthylsilyle, 2-triméthylsilyl-éthanesulfonyle, trityle et groupes trityle substitués, allyloxycarbonyle, 9-fluorenylméthyloxycarbonyle, ou nitro-vératryloxycarbonyle ;
ou, en variante, les deux R^{c} liés au même atome d'azote sont pris conjointement avec cet atome d'azote pour former un hétérocycloalkyle ou hétéroaryle à 5 à 8 membres qui peut optionnellement inclure un ou plusieurs des mêmes ou de différents hétéroatomes supplémentaires et qui peut optionnellement être substitué avec un ou plusieurs des mêmes ou de différents groupes R^{a} ;
R²¹, R²² et R²³ sont chacun, indépendamment les uns des autres, sélectionnés parmi hydrogène ou phosphonooxyalkyle ;
R²⁴ est sélectionné parmi hydrogène, alkyle (C₁-C₈), ou phosphonooxyalkyle ;
chaque *m* est, indépendamment des autres, un nombre entier de 1 à 3 ; et
chaque n est, indépendamment des autres, un nombre entier de 0 à 3.

2. Composé pour l'utilisation selon la revendication 1, dans lequel au moins un de : R²¹, R²², R²³, et R²⁴ est phosphonooxyalkyle.

3. Composé pour l'utilisation selon la revendication 1 ou la revendication 2, dans lequel au moins un de : R²¹, R²², R²³, et R²⁴ est hydrogène.

4. Composé pour l'utilisation selon l'une quelconque des revendications 1 à 3, dans lequel R²¹ est phosphonooxyalkyle, et R²², R²³, et R²⁴ sont hydrogène.

5. Composé pour l'utilisation selon l'une quelconque des revendications 1 à 4, dans lequel le composé a une formule sélectionnée parmi 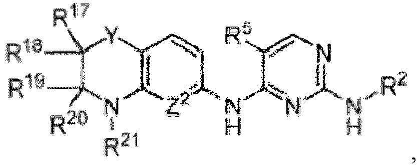 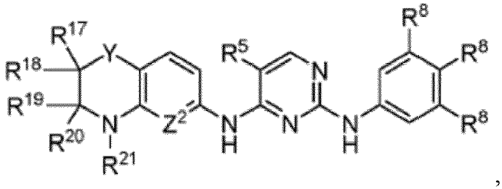 ou 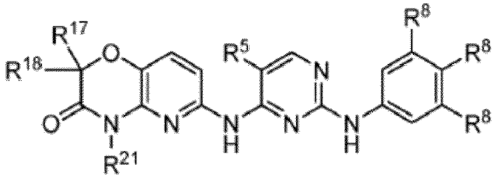 ou un sel, solvate, N-oxyde ou promédicament de celui-ci.

6. Composé pour l'utilisation selon l'une quelconque des revendications 1 à 5, dans lequel le composé a une formule 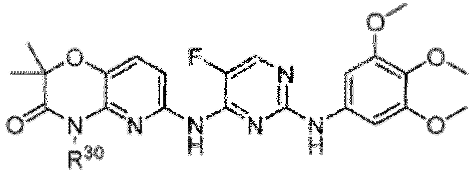 ou un sel, solvate, N-oxyde ou promédicament de celui-ci, où R³⁰ est H ou phosphonooxyalkyle.

7. Composé pour l'utilisation selon l'une quelconque des revendications 1 à 6, dans lequel le composé 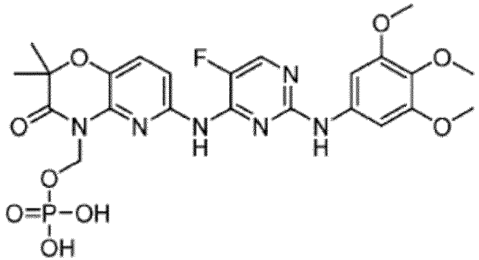 ou un sel et/ou solvate de celui-ci.

8. Composé pour l'utilisation selon l'une quelconque des revendications 1 à 7, dans lequel le composé

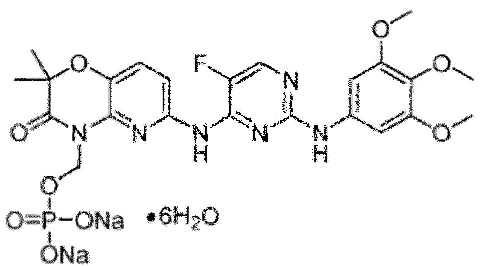

9. Composé pour l'utilisation selon l'une quelconque des revendications 1 à 6, dans lequel le composé

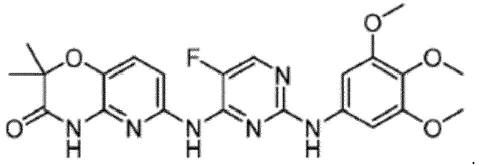

10. Composé pour l'utilisation selon l'une quelconque des revendications 1 à 9, dans lequel l'administration du composé améliore un signe ou symptôme de CRS, par rapport à la sévérité du signe ou du symptôme avant l'administration du composé ;
de préférence dans lequel le signe ou le symptôme est une fièvre.

11. Composé pour l'utilisation selon l'une quelconque des revendications 1 à 10, dans lequel l'administration comprend :
l'administration à un sujet auquel a auparavant été administrée une première thérapie pour laquelle le CRS est un effet secondaire connu, suspecté, ou potentiel ; ou
l'administration à un sujet auquel sera, ou est simultanément, administrée une première thérapie pour laquelle le CRS est un effet secondaire connu, suspecté, ou potentiel.

12. Composé pour l'utilisation selon la revendication 11, dans lequel la première thérapie comprend une thérapie cellulaire ;
de préférence dans lequel la thérapie cellulaire comprend une thérapie à expression de récepteur antigénique chimérique (RAC), une thérapie à récepteur transgénique, ou une association de celles-ci.

13. Composé pour l'utilisation selon l'une quelconque des revendications 1 à 12, dans lequel l'administration du composé comprend en outre l'administration d'un second agent thérapeutique ;
de préférence dans lequel le second agent thérapeutique est un stéroïde, un agent anti-inflammatoire, un immunosuppresseur, ou une association de ceux-ci.

14. Composé pour l'utilisation selon la revendication 13, dans lequel :
le stéroïde est alclométhasone, algestone, beclométhasone, bétaméthasone, budésonide, clobétasol, clobétasone, clocortolone, cloprednol, corticostérone, cortisone, cortivazol, déflazacort, désonide, désoximéthasone, dexaméthasone, diflorasone, diflucortolone, difluprednate, énoxolone, fluazacort, flucloronide, fludrocortisone, fluméthasone, flunisolide, fluocinolone, fluocinonide, fluocortine, fluocortolone, fluorométholone, flupérolone, fluprednidène, fluprednisolone, flurandrénolide, fluticasone, formocortal, halcinonide, halobétasol, halométasone, haloprédone, hydrocortamate, hydrocortisone, étabonate de lotéprednol, maziprédone, médrysone, meprednisone, méthylprednisolone, mométasone, paraméthasone, prednicarbate, prednisolone, prednisone, prednival, prednylidène, rimexolone, tixocortol, triamcinolone, ou une quelconque association de ceux-ci ;
l'agent anti-inflammatoire est un aminosalicylate, inhibiteur de cyclooxygénase, diclofénac, étodolac, famotidine, fénoprofène, flurbiprofène, kétoprofène, kétorolac, ibuprofène, indométhacine, méclofénamate, acide méfénamique, meloxicam, nambumétone, naproxène, oxaprozine, piroxicam, salsalate, sulindac, tolmétine, ou une association de ceux-ci ; ou
l'immunosuppresseur est mercaptopurine, un corticostéroïde, un agent alkylant, un inhibiteur de calcineurine, un inhibiteur d'inosine monophosphate déshydrogénase (EMPDH), un agent conçu pour supprimer l'immunité cellulaire tout en laissant la réponse immunologique humorale du receveur intacte, ou une association de ceux-ci.

15. Composé pour l'utilisation selon la revendication 13, dans lequel le second agent thérapeutique est dexaméthasone ou prednisone, ou une association de celles-ci.
